# FASCICULE DE BREVET EUROPEEN

(11) **EP 2 999 467 B1**
(45) Date de publication et mention de la délivrance du brevet: **08.01.2020**
(21) Numéro de dépôt: 14729418.5
(22) Date de dépôt: 13.05.2014
(51) Int. Cl.: A61K 31/352, A61K 31/202, A61P 19/08, A61K 45/06, A23L 33/10, A23L 33/115

(54) **UTILISATION D'UNE ASSOCIATION DE DEUX COMPOSÉS POUR LE TRAITEMENT ET/OU LA PRÉVENTION DE TROUBLES OSSEUX**
VERWENDUNG EINER KOMBINATION VON ZWEI VERBINDUNGEN ZUR BEHANDLUNG UND/ODER PRÄVENTION VON KNOCHENERKRANKUNGEN
USE OF A COMBINATION OF TWO COMPOUNDS FOR THE TREATMENT AND/OR PREVENTION OF BONE DISORDERS

(30) Priorité: 13.05.2013 FR 1354275
(43) Date de publication de la demande: 30.03.2016
(73) Titulaire: Institut National de la Recherche Agronomique (INRA), 75007 Paris (FR)
(72) Inventeur: LEOTOING, Laurent, F-63540 Romagnat (FR); COXAM, Véronique, F-63122 Ceyrat (FR); WITTRANT, Yohann, F-63450 Chanonat (FR)
(74) Mandataire: Jacobacci Coralis Harle
(86) Numéro de dépôt international: PCT/FR2014/051108
(87) Numéro de publication internationale: WO 2014/184484

(56) Documents cités:
- WO-A2-2012/159092
- GB-A- 2 458 467
- SIK-WON CHOI ET AL: "Fisetin Inhibits Osteoclast Differentiation via Downregulation of p38 and c-Fos-NFATc1 Signaling Pathways", EVIDENCE-BASED COMPLEMENTARY AND ALTERNATIVE MEDICINE, vol. 273, no. 5279, 1 janvier 2012 (2012-01-01), pages 1236-9, XP055092772, ISSN: 1741-427X, DOI: 10.1074/jbc.M408795200
- A. CASADO-DÍAZ ET AL: "The omega-6 arachidonic fatty acid, but not the omega-3 fatty acids, inhibits osteoblastogenesis and induces adipogenesis of human mesenchymal stem cells: potential implication in osteoporosis", OSTEOPOROSIS INTERNATIONAL, vol. 24, no. 5, 27 octobre 2012 (2012-10-27), pages 1647-1661, XP055092795, ISSN: 0937-941X, DOI: 10.1007/s00198-012-2138-z

### SEQUENCE LISTING

<110> Institut National de la Recherche Agronomique INRA
<120> Utilisation d'une association de deux composés pour el traitement et/ou la prévention de troubles osseux
<130> Z055PCT/EP
<140> wo2014/187784
   <141> 2014-05-13
<150> FR13/54275
   <151> 2013-05-13
<160> 8
<170> PatentIn version 3.5
<210> 1
   <211> 19
   <212> DNA
   <213> artificial sequence
<220>
   <223> ALP F 5'-3'
<400> 1
   ggccagctac accacaaca 19
<210> 2
   <211> 24
   <212> DNA
   <213> artificial sequence
<220>
   <223> ALP R 5'-3'
<400> 2
   ctgagcgttg gtgttatatg tctt 24
<210> 3
   <211> 18
   <212> DNA
   <213> artificial sequence
<220>
   <223> OCN F 5'-3'
<400> 3
   agactccggc gctacctt 18
<210> 4
   <211> 20
   <212> DNA
   <213> artificial sequence
<220>
   <223> OCN R 5'-3'
<400> 4
   caagcagggt taagctcaca 20
<210> 5
   <211> 21
   <212> DNA
   <213> artificial sequence
<220>
   <223> COL1 F 5'-3'
<400> 5
   catgttcagc tttgtggacc t 21
<210> 6
   <211> 20
   <212> DNA
   <213> artificial sequence
<220>
   <223> COL1 R 5'-3'
<400> 6
   gcagctgact tcagggatgt 20
<210> 7
   <211> 20
   <212> DNA
   <213> artificial sequence
<220>
   <223> GAPDH F 5'-3'
<400> 7
   aactttggca ttgtggaagg 20
<210> 8
   <211> 20
   <212> DNA
   <213> artificial sequence
<220>
   <223> GAPDH R 5'-3'
<400> 8
   ggatgcaggg atgatgttct 20

## Description

### DOMAINE DE L'INVENTION

L'invention se rapporte au domaine des troubles liés à une diminution de la synthèse osseuse et/ou à une augmentation de la résorption osseuse, et notamment aux pathologies osseuses, et à leur prévention et/ou leur traitement. Elle concerne notamment des associations présentant une activité améliorée sur ces phénomènes et leur utilisation, en particulier sous forme d'apport nutritionnel ou de composition pharmaceutique.

### ETAT DE LA TECHNIQUE

L'os n'est pas un tissu statique. Il subit un remodelage constant par destruction et synthèse *de novo* du tissu osseux dans un processus complexe impliquant deux types principaux de cellules, respectivement les ostéoblastes, qui produisent du tissu osseux nouveau, et les ostéoclastes qui résorbent l'os.

Les activités de ces cellules sont régulées par un grand nombre de cytokines et de facteurs de croissance, dont la plupart ont été identifiés et clonés comme cela est décrit dans la revue générale de Mundy (Mundy, G.R., 1996, Clin. Orthop., vol.324 :24-28 ; Mundy, G.R., 1993, J.Bone Miner Res, vol.8 : S 505 - S 510).

Les ostéoblastes, cellules responsables de la formation de l'os, se différencient à partir de cellules précurseurs et expriment et secrètent de nombreuses enzymes et protéines de structure de la matrice de l'os, y compris le collagène de type I, l'ostéocalcine, l'ostéopontine et la phosphatase alcaline (Stein G. et al., 1990, Curr. Opin Cell Biol. vol.2: 1018-1027; Harris S et al., 1994, J. Bone Miner Res Vol.9:855-863).

Les ostéoblastes synthétisent aussi de nombreux peptides régulateurs de croissance, y compris les peptides BMPs (pour "Bone Morphogenetic Proteins »), qui sont stockés dans la matrice osseuse, et qui sont vraisemblablement responsables de la formation normale de l'os.

Comme la phosphatase alcaline, l'ostéocalcine et l'ostéopontine, les peptides BMPs sont exprimés par des ostéoblastes en culture au moment où ces cellules prolifèrent et se différencient.

Les ostéoclastes sont des cellules multinucléées qui sont responsables de la perte osseuse, dans un processus communément désigné la résorption osseuse.

Chez un adulte en bonne santé, homme ou animal, l'action conjointe des ostéoblastes et des ostéoclastes assure un remodelage du tissu osseux par résorption et synthèse de novo de l'os qui permet le maintien de la masse osseuse. Chez un sujet adulte sain, la vitesse de formation des ostéoclastes et des ostéoblastes est telle qu'il y a un équilibre entre la formation de l'os et la résorption osseuse. La masse osseuse se constitue progressivement entre la naissance et l'âge de 18-20 ans, jusqu'à une quantité maximale que l'on appelle le pic de masse osseuse.

Chez l'homme ou l'animal, il existe de nombreux états caractérisés par la nécessité d'accroître la formation osseuse. Par exemple, dans le cas de fractures osseuses, il est impératif de stimuler la croissance osseuse, afin d'accélérer une réparation complète de l'os. Ce besoin est également identifié dans les maladies dentaires et parodontales, les maladies tumorales ostéolytiques telles que la majorité des tumeurs osseuses primaires et secondaires (lésions métastatiques de l'os) et les maladies ostéolytiques. On recherche également la stimulation d'une croissance osseuse dans le cas d'hyperparathyroïdisme primaire et secondaire, ainsi que dans l'ostéoporose associée au diabète ou liées à la prise de glucocorticoïdes.

Par ailleurs, on observe une hétérogénéité importante dans la valeur du pic de masse osseuse selon les individus, en raison des variations dans le processus de croissance osseuse dès le plus jeune âge.

En outre, la diminution de la masse osseuse se passe différemment chez la femme et l'homme. Chez l'homme, la mise en place d'une ostéopénie est régulière, à raison de 0,5% par an. Chez la femme, au moment de la ménopause, une accélération importante de la perte osseuse se produit : 3 à 5% pendant 2 à 3 ans environ, puis 1 à 2 % pendant les 5 à 10 ans suivants. Cette perte osseuse appelée « post-ménopausique » est due à un emballement des ostéoclastes lié à la baisse de l'imprégnation oestrogénique, consécutive à la ménopause. Ainsi, au final, la femme va perdre 30 à 50 % de sa masse osseuse (selon le site squelettique), au cours de la vie. Par conséquent, les individus possédant initialement une valeur faible de pic de masse osseuse risquent d'atteindre plus rapidement le seuil fracturaire. En outre, le phénomène d'ostéopénie lié à l'âge peut être aggravé par des facteurs environnementaux tels que notamment une alimentation carencée en calcium et en vitamine D.

Chez l'homme et les autres mammifères, une grande diversité de troubles sont également liés à un métabolisme anormal de la résorption osseuse ou de la formation de l'os, entraînant un déséquilibre du métabolisme ou remodelage osseux (déséquilibre du rapport entre la formation et la résorption osseuse), ou simplement à une diminution de la masse osseuse associée au vieillissement.

Parmi les désordres pathologiques, ou les troubles, associés à un déséquilibre du métabolisme osseux, on citera notamment les troubles, ou les pathologies, tels que l'ostéoporose, la maladie de Paget, la perte osseuse ou l'ostéolyse observée à proximité d'une prothèse, les maladies dentaires et parodontales, les maladies tumorales ostéolytiques telles que la majorité des tumeurs osseuses primaires et secondaires (lésions métastatiques de l'os), les maladies ostéolytiques l'hypercalcémie due à un cancer.

Parmi les troubles liés à une augmentation de la résorption osseuse, le plus commun est l'ostéoporose, dont la manifestation la plus fréquente est observée chez les femmes, après le début de la ménopause. L'ostéoporose est une maladie squelettique systémique chronique résultant d'une altération, principalement liée à l'âge, de l'architecture trabéculaire et d'une réduction du contenu minéral de l'os, engendrant une fragilisation du squelette et une augmentation du risque de fracture. Chez les individus ostéoporotiques, il se produit un déséquilibre dans le processus de remodelage osseux aboutissant à une perte d'os qui se fait à une vitesse plus rapide que sa vitesse de formation.

Certains des troubles ou pathologies du métabolisme osseux peuvent également apparaître après une immobilisation de longue durée, par exemple une hospitalisation prolongée, après une paralysie ou une hémiplégie ou encore un séjour en apesanteur.

Il existe enfin d'autres facteurs susceptibles d'accroître la perte osseuse conduisant à l'ostéoporose, telle que la consommation de cigarettes, l'abus d'alcool, un mode de vie sédentaire, un faible apport en calcium, une alimentation déséquilibrée ou encore une carence en vitamine D.

Ainsi, quel que soit l'individu, un traitement préventif précoce stimulant la formation osseuse, ou limitant la perte osseuse, est préconisé, de manière à réduire le plus possible les déséquilibres du remodelage osseux, qui se produisent de manière spontanée avec le vieillissement, notamment les risques de fractures osseuses précoces. En outre, du fait que l'ostéoporose, comme d'autres troubles associés à une perte osseuse, constitue un trouble chronique, sa prévention et son traitement doivent être envisagés sur une longue durée et ne doit donc plus être restreinte à l'individu âgé.

Il est ainsi couramment admis, qu'une prise en charge précoce doit être privilégiée puisque les deux phases critiques pour le capital osseux sont :
- la période de croissance permettant l'acquisition de la masse osseuse maximale (pic de masse osseuse) ;
- le vieillissement conditionnant la vitesse de perte de la masse osseuse.

La prévalence des pathologies associées au vieillissement s'est fortement accrue avec l'augmentation de l'espérance de vie. Parmi les manifestations de la sénescence, l'atteinte de l'appareil locomoteur est particulièrement coûteuse, invalidante, et accélère considérablement l'entrée en dépendance. Ainsi, les pathologies osseuses, dont l'ostéoporose, constituent un problème majeur de santé publique.

A titre d'exemple, les coûts engendrés par la fracture de l'extrémité supérieure de la hanche dépasseraient 9.10⁹ euros en Europe (Rapport de la Communauté Européenne, 1999). Par ailleurs, seuls 25 % des patients maintiennent une qualité de vie équivalente à celle antérieure à l'intervention chirurgicale et environ 30 % décèdent dans l'année consécutive. Chez les populations caucasiennes après 50 ans, le risque cumulé sur les années restant à vivre de développer le syndrome ostéoporotique atteint 40 % chez la femme et 13 % chez l'homme. Au-delà de 80 ans, 70% des personnes sont ostéoporotiques.

Il existe un certain nombre de composés actifs sur la stimulation de la formation de l'os ou l'inhibition de la résorption osseuse, parmi lesquels on peut citer la famille des polyphosphonates (brevet européen EP n°210 728), les oxazolidinones thioamides (demande de brevet US 2002/0010341), les amino bis-phosphonates (demande de brevet US 2001/0046977), les isoflavones (demande de brevet US 2002/0035074). On a également proposé l'utilisation d'extrait d'une plante du genre Lycopersicon (demande de brevet US 2002/0009510) ou de l'héspéridine, un flavanone glycosylé présent dans les agrumes (WO 2004002496). La fisétine est connue pour sa capacité d'inhiber la formation d'ostéoclastes induite par RANKL (SIK-WON CHOI ET AL: "Fisetin Inhibits Osteoclast Differentiation via Downregulation of p38 and c-Fos-NFATc1 Signaling Pathways", EVIDENCE-BASED COMPLEMENTARY AND ALTERNATIVE MEDICINE, vol. 273, no. 5279, 2012, pages 1236-9).

L'utilisation d'acides gras poly insaturés, pour améliorer la santé et prévenir la perte osseuse a été proposée notamment par Sun D et al. (J. Bone Miner. Res., 2003), Rahman MM et al. (J. Cell Physiol., 2008), Wauquier et al. (J. Bone, 2011), Farina et al. (Am. J. of Clinical Nutrition, 2011). Dans la demande internationale WO 02074308, un acide gras poly insaturé est proposé seul ou en association avec un composé isoflavone, dans le cadre de la prévention de l'ostéoporose.

Des compositions enrichies en dérivés de la vitamine D₃ et en glycosides de flavonols, ou comprenant une association de nombreux polyphénols ont également été proposées pour le traitement de pathologies impliquant une perte osseuse, comme l'ostéopénie ou l'ostéoporose (WO 2009130005), ou associées à une déficience oestrogénique (WO 200982459).

La demande EP 1127572 décrit également l'utilisation des flavones, dans le traitement de pathologies nécessitant l'inhibition de la cycloxygénase 2, comme la maladie d'alzheimer et l'arthrite.

Des formulations, comprenant une association de fisétine et de DHA, ont été décrites dans le cadre d'applications thérapeutiques totalement différentes, comme le traitement des maladies neuro-dégénératives (WO 2012159092) et des maladies cardiovasculaires (US20100021573).

Malgré ces connaissances, l'arsenal thérapeutique à la disposition des médecins reste peu étendu. En particulier, les pouvoirs publics recommandent désormais que l'hormonothérapie substitutive qui était jusqu'à présent le traitement de référence de l'ostéoporose, ne soit plus prescrite en première intention, entrainant ainsi un vide thérapeutique conséquent. Les stratégies actuelles de prévention nutritionnelle de l'ostéoporose reposent exclusivement sur une supplémentation calcium/vitamine D.

Il est donc souhaitable de disposer d'alternatives thérapeutiques, dépourvues d'effets secondaires, dont l'efficacité est avérée, et la mise en œuvre simple. Un tel traitement pourra ainsi aisément être intégré dans le cadre d'une approche prophylactique ostéoprotectrice ou d'un traitement chronique.

Il serait également avantageux de disposer de traitement peu couteux, pour lesquels la fabrication, ou le stockage, est simple et implique des matières premières peu onéreuses et disponibles en quantité suffisantes.

### RESUME DE L'INVENTION

Ces buts et d'autres sont atteints par la présente invention. En effet, il a été démontré, dans le cadre de la présente invention, que l'association de la fisétine et du DHA présente une action synergique (significativement supérieure à la simple addition des effets individuels) sur l'inhibition de la perte osseuse. Ces résultats démontrent pour la première fois une synergie d'action de ces deux molécules sur les mécanismes de la perte osseuse, ouvrant ainsi de nouvelles perspectives de traitement et/ou de prévention nutritionnelle des troubles liés à un déséquilibre du rapport entre formation et résorption osseuse. Ces associations, ou compositions, sont utiles pour le traitement et/ou la prévention de pathologies osseuses.

L'invention est définie par les revendications. Tout objet ne relevant pas du champ d'application des revendications est mentionné à titre d'information uniquement.

L'invention se rapporte donc à une association comprenant au moins (i) la fisétine et (ii) le DHA, ou l'un de ses dérivés choisis sous forme d'un glycéride ou d'un phospholipide, en tant que principes actifs, ou à une composition la contenant, pour son utilisation pour le traitement et/ou la prévention de troubles associés à une perte osseuse, et/ou à un déséquilibre du rapport entre la formation et la résorption osseuse.

En particulier, une telle association, ou la composition la contenant, peut être utilisée pour inhiber la perte osseuse.

Il a également été découvert dans le cadre de l'invention, que la fisétine présente une action stimulatrice sur la différentiation des ostéoblastes, concourant ainsi activement à la formation osseuse. La fisétine peut donc être utilisée pour stimuler la formation osseuse.

Dans certains modes de réalisation de l'invention, l'association de fisétine et de DHA, et/ou d'un des dérivés du DHA choisis sous forme d'un glycéride ou d'un phospholipide, telle que décrite précédemment, est donc également utilisée pour stimuler la formation osseuse, en particulier pendant la croissance ou dans le traitement, et/ou la prévention, de pathologies nécessitant une stimulation de la formation osseuse.
Les troubles, associés à une perte osseuse et/ou à un déséquilibre du rapport entre la formation et la résorption osseuse comprendront notamment la perte osseuse associée au vieillissement et/ou à la ménopause, la perte osseuse consécutive à des maladies métaboliques, telles que le diabète ou l'obésité massive, et/ou à la prise de certains médicaments comme les corticoïdes, l'ostéolyse observée à proximité d'une prothèse, la perte osseuse après une intervention chirurgicale ou encore d'une maladie pathologie dentaire, la maladie de Paget, les maladies tumorales ostéolytiques, telles que la majorité des tumeurs osseuses primaires et secondaires (notamment les lésions métastatiques de l'os), les maladies ostéolytiques, et l'hypercalcémie due à un cancer, ou encore après fracture.

De façon générale également, l'association selon l'invention, ou une composition la contenant, est adaptée à la prévention, et/ou au traitement de l'ostéopénie, la raréfaction osseuse, la fragilisation du tissu osseux, ou de l'ostéoporose.

La fisétine, et/ou le DHA, et/ou l'un au moins des dérivés du DHA choisis sous forme d'un glycéride ou d'un phospholipide, peuvent être sous forme plus ou moins purifiés, et notamment sous forme d'extraits naturels en contenant. L'utilisation de tels extraits permet, la mise en œuvre de traitements entièrement naturels, sains et dépourvus d'effets secondaires nocifs.

Selon un aspect particulier, dans une association selon l'invention, ou dans une composition la contenant, la fisétine et le DHA (ou l'un de ses dérivés) sont présents suivant des ratios (poids/poids) Fisétine/DHA allant de 100/1 à 1/100. Une telle gamme de ratio comprendra notamment les ratios 100/1, 50/1, 20/1, 10/1, 4/1, 2/1, 1/1, 1/10, 1/20 1/50 et 1/100.

Dans certains modes de réalisation de l'invention, la fisétine est administrée à une dose quotidienne de 0.01 à 1 g/jour et le DHA, ou l'un de ses dérivés est administré à une dose quotidienne de 0.01 à 3 g/jour.

L'association de la fisétine et du DHA, et/ou des dérivés du DHA choisis sous forme d'un glycéride ou d'un phospholipide, selon l'invention, peut être sous forme d'un produit de combinaison comprenant au moins :
i) une quantité efficace de fisétine et
ii) une quantité efficace de DHA ou de l'un de ses dérivés;
pour une utilisation séquentielle, séparée ou simultanée de l'une avec l'autre dans le temps.

Dans un mode de réalisation de l'invention, l'association, est sous forme d'au moins une composition comprenant au moins un excipient physiologiquement acceptable.

Plus particulièrement, l'association peut être sous forme d'une composition unique comprenant en outre au moins un excipient physiologiquement acceptable.

Alternativement, l'association peut être sous forme d'au moins deux compositions a) et b) comprenant respectivement au moins (i) la fisétine et (ii) le DHA ou l'un de ses dérivés ; au moins une de ces compositions comprenant au moins un excipient physiologiquement acceptable.

Typiquement, une composition selon l'invention est adaptée à une administration par voie orale.

L'association, ou la composition la contenant, selon l'invention peut selon certains modes de réalisation être sous forme de complément nutritionnel ou alimentaire. La ou les composition(s) de l'invention peuvent notamment se présenter sous forme(s) de gélule(s), poudre(s), lyophilisat(s), granule(s), comprimé(s), capsule(s), tablette(s), pilules(s), chewing-gum(s), ampoule(s), sirop(s), infusion(s), jus, macérât(s), extrait sec, extrait mou, extrait hydro-alcoolique.

Dans un mode de réalisation particulier, l'association de l'invention, ou la composition la contenant, se présente sous forme de produits laitiers, dérivés de lait végétaux, produits dérivés de fruits ou légumes, de produits céréaliers, de corps gras, de dérivés issus de produits de la mer, notamment de dérivés de poissons, de boissons, ou de plats cuisinés.

Dans un mode de réalisation particulier, l'association de l'invention, ou la composition la contenant contient, outre les ingrédients actifs (i) et (ii) décrits précédemment, au moins un autre composé choisi parmi le groupe constitué par les sels de calcium inorganiques, les composés contenant du calcium, les vitamines, les éléments minéraux, les fibres, les prébiotiques, les composés source d'énergie tels que notamment les glucides, les protéines ou les acides aminés.

Sauf s'il en est disposé explicitement autrement, les différents modes de réalisations de l'invention, décrits dans la présente demande, pourront être pris individuellement ou en combinaison les uns avec les autres.

### DESCRIPTION DES FIGURES

Figure 1 : Effet synergique de l'association fisétine (5 µM) et du DHA (50 µM) sur l'inhibition *in vitro* de la différentiation des ostéoclastes. 1A :
Figure 2 : Effet de la fisétine sur la différentiation des ostéoblastes *in vitro.*
Figure 3 : Effet de la fisétine *in vivo* sur la perte osseuse.

### DESCRIPTION DETAILLEE DE L'INVENTION

La présente invention se rapporte à une association comprenant au moins la fisétine et le DHA ou l'un de ses dérivés choisis sous forme d'un glycéride ou d'un phospholipide, pour son utilisation dans le traitement et/ou la prévention de pathologies, ou de troubles osseux

En effet, la demanderesse a démontré, que l'association de la fisétine et du DHA a un effet synergique sur l'inhibition de la différentiation des ostéoclastes. Notamment, et comme illustré dans les exemples de la présente demande, l'association Fisétine/DHA a un effet significativement supérieur sur l'inhibition de la différentiation des ostéoclastes, à celui résultant de l'addition des effets de la fisétine ou du DHA administrés individuellement. Une telle association sera donc particulièrement utile pour inhiber la résorption osseuse, chez l'homme ou l'animal.

La demanderesse a également découvert, que la fisétine stimule la différentiation et l'activité des ostéoblastes, via l'activation du facteur de transcription Runx2 et promeut ainsi directement la formation osseuse. La fisétine, et en particulier, l'association comprenant au moins la fisétine, et le DHA, ou l'un de ses dérivés, peut donc également être utilisée, dans certains modes de réalisation pour stimuler la formation osseuse.

Avantageusement une telle association est donc destinée à traiter ou prévenir les troubles associés à une perte osseuse pathologique, et/ou à un déséquilibre du métabolisme osseux.

Dans certains modes de réalisation, la fisétine, et le DHA, ou l'un de ses dérivés, sont présents dans l'association de l'invention (ou dans la composition la contenant) suivant un ratio (poids/poids) fisétine/DHA variant de 100/1 à 1/100, en particulier de 50/1 à 1/50, de 20/1 à 1/50, de 10/1 à 1/50, de 10/1 à 1/10, de 1/1 à 1/50, ou de 1/1 à 1/10.

Selon certains modes de réalisation, la fisétine associée au DHA ou l'un de ses dérivés, tel que précédemment décrit, est utilisée comme complément nutritionnel et notamment, est présente dans une composition à usage nutritionnel pour l'homme ou l'animal, notamment un complément alimentaire ou un aliment.

Selon d'autres modes de mise en œuvre de l'invention, la fisétine associée avec le DHA ou l'un de ses dérivés, tel que précédemment décrit, est sous forme d'une composition pharmaceutique, à usage humain ou vétérinaire.

Typiquement les compositions selon l'invention comprennent en outre au moins un excipient physiologiquement acceptable.

Dans un mode de réalisation particulier, l'association selon l'invention, ou la composition la contenant comprendra uniquement en tant que principes actifs, la fisétine et le DHA et/ou l'un de ses dérivés choisis sous forme d'un glycéride ou d'un phospholipide. Plus particulièrement encore les principes actifs (i) et (ii) consisteront respectivement en la fisétine et le DHA.

La fisétine, ou fisétol, ou 3,3',4',7-tétrahydroxyflavone est un composé organique de la famille des flavonols, une sous-famille des flavonoïdes. Les flavonols forment un sous-groupe de flavonoïdes dérivés de la 3-hydroxyflavone (3-hydroxy-2-phénylchromén-4-one en nomenclature IUPAC) ou flavonol, c'est-à-dire des flavonoïdes possédant un hydroxyle phénolique en C3 et une fonction carbonyle C=O en C4 sur l'hétérocycle central du squelette de base des flavonoïdes. Ce sont des pigments végétaux de couleur jaune, plus ou moins clairs. Ils diffèrent par le nombre et la position d'hydroxyle phénolique -OH, parfois méthylés (groupes méthoxy).

Parmi les flavonols, on peut citer de manière non limitative, le quercétol, le kaempférol, le myricétol, l'azaléatine, la galangine, la gossypétine, l'herbécatine, l'isorhamnétine, la morine, la myricétine, la natsudaidaine, le pachypodol, la rhamnazine, la rhamnétine, la fisétine.

La fisétine possède la structure chimique de la formule (I) suivante : 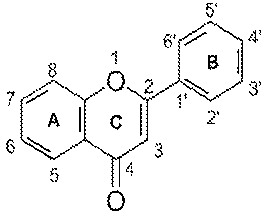 dans laquelle, les atomes de carbone en position 7, 3, 3' et 4' sur les cycles respectifs A, C et B sont substitués par un groupement hydroxy (-OH). La fisétine possède donc la structure de formule (II) ci-dessous :

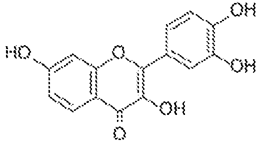

Une action bénéfique de la fisétine a également été décrite dans le diabète (Maher P, et al., 2013), le cancer (Adhami VM, Syed DN, Khan N, Mukhtar H., Biochem Pharmacol. 2012 Nov 15;84(10):1277-81. Epub 2012 Jul 25), et dans son utilisation en tant qu'anti-oxydant (Khan N, Syed DN, Ahmad N, Mukhtar H., Antioxid Redox Signal. 2012) et/ou anti-inflammatoire (Prakash D, Gopinath K, Sudhandiran G., Neuromolecular Med. 2013 Mar;15(1):192-208. Epub 2013 Jan 13).

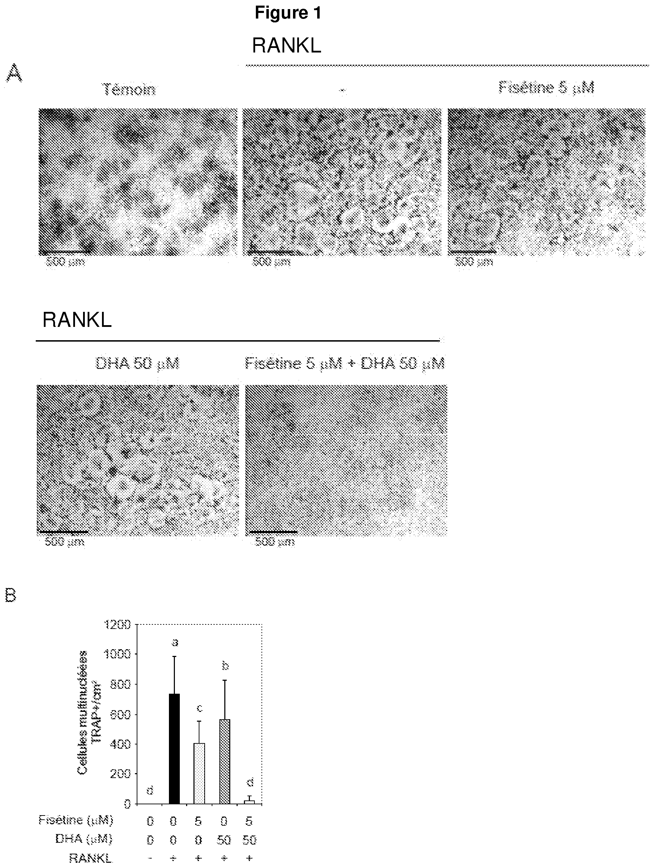

Par dérivé de la fisétine, on entend les isomères, les précurseurs, les métabolites de la fisétine ainsi que les analogues naturels ou synthétiques de la fisétine. On entend notamment les formes solvatées (et notamment hydratées), et/ou méthylées, et/ou méthoxylées , et/ou éthoxylées, et/ou sulfatées et/ou glycosylées (en particulier les hétérosides et notamment les glucuronosides) de la fisétine ainsi que leurs sels physiologiquement acceptables. Les dérivés de la fisétine sont tous physiologiquement acceptables et possèdent une activité biologique similaire à la fisétine. Typiquement l'activité biologique d'un dérivé de la fisétine peut être évaluée comme illustré dans les résultats de la présente demande. En particulier, un dérivé de la fisétine présente, en association avec le DHA ou l'un de ses dérivés, une activité synergique limitant la différentiation des ostéoclastes en culture (voir figure 1) au moins égale à celle de la fisétine. De préférence, font partie de l'invention, la fisétine et ses formes solvatées et notamment hydratées; ainsi que leurs sels physiologiquement acceptables. Typiquement, la fisétine est sous forme solvatée et/ou sous forme d'un sel physiologiquement acceptable.

Dans certains modes de réalisation, les dérivés de la fisétine ne possèdent pas de groupement hydroxy substitué sur le cycle A. Ces composés peuvent présenter en positions 3', 4' ou 3', 6' du cycle B de la formule (I) des groupes substitués choisis parmi les groupe hydroxy, méthoxy et/ou éthoxy, et la position 3 sur le cycle C peut être ou non substituée par un groupe hydroxy.

On retiendra à titre illustratif, les dérivés suivants de la fisétine : la dihydrofisétine (ou fustine), ainsi que la fisétine glucoside, comme la fisétine 8-C-glucoside, la fisétine 4-O-glucoside, la fisétine 3-glucoside, la fisétine 4'-glucoside, la fisétine 7-glucoside, la fisétine 3,7-diglucoside ; la fisétine 7-rutinoside, la fisétine 3-methyl ether, la fisétine 4'-méthyl éther, la fisétine 3, 3', 4', 7- tétraméthyléther, la Fisétinidine, le géraldol, la 3, 3', 4'-trihydroxyflavone, la 6, 7 diméthyl 3', 4' diéthoxylflavone, la 6,7-diméthyl 3 hydroxy 3',4'diméthoxyflavone, la 6,7-diméthyl, 3, 3', 4' trihydroxyflavone, la 6,7-diméthyl, 3', 4' trihydroxyflavone, la 6,7-diméthyl, 3'-méthoxy 4'-hydroxyflavone, la 6,7-diméthyl, 3'-méthoxy 3, 4'-dihydroxyflavone, la 6,7-diméthyl, 4'-méthoxy 3, 3'-dihydroxyflavone, la 6,7-diméthyl, 3, 4', 6'-trihydroxyflavone, la 6,7-diméthyl, 4', 6'-dihydroxyflavone.

Selon l'invention la fisétine, pourra être sous forme plus ou moins purifiée, obtenue par synthèse chimique, ou purifiée à partir de produits naturels. Elle pourra ainsi se trouver sous la forme d'un ou plusieurs extraits d'un ou plusieurs produits naturels en contenant.

De façon non limitative, et à titre d'exemple, la fisétine selon l'invention pourra être obtenue à partir de fraise, de mangue, de kiwi, de pêche, de kaki, de pomme, de tomate, d'oignons, de concombre, de certains arbres du genre Acacia, (*Acacia greggii, Acacia berlandieri*), de l'arbre Quebracho colorado, du cyprès de Nootka (*Xanthocyparis nootkatensis*), de fleurs Butea monosperma (palash, flamme de la forêt), ou de plantes du genre Rhus.

L'acide docosahexaénoïque (DHA) est un acide gras polyinsaturé oméga-3 à 22 atomes de carbone et contenant 6 doubles liaisons « carbone-carbone », correspondant à l'acide tout-cis-Δ 4,7,10,13,16,19 acide hexa-enoique, ou noté 22:6n-3.

L'utilisation du DHA a été décrite dans le cadre du traitement ou de la prévention de la dépression (Kang JX, Gleason ED., CNS Neurol Disord Drug Targets. 2013 Apr 4.), du cancer (Zhang G, Panigrahy D, Mahakian LM, Yang J, Liu JY, Stephen Lee KS, Wettersten HI, Ulu A, Hu X, Tam S, Hwang SH, Ingham ES, Kieran MW, Weiss RH, Ferrara KW, Hammock BD., Proc Natl Acad Sci U S A. 2013 Apr 16;110(16):6530-5. Epub 2013 Apr 3), de l'insulino-résistance et des troubles associés comme des troubles de l'attention et pour la promotion du développement cérébral et cognitif (Transler C, Eilander A, Mitchell S, van de Meer N., J Atten Disord. 2010 Nov;14(3):232-46. Epub 2010 Apr 27. Review; Schuchardt JP, Huss M, Stauss-Grabo M, Hahn A, Eur J Pediatr. 2010 Feb;169(2):149-64. Epub 2009 Aug 12.), des pathologies inflammatoires, comme l'arthrite rhumatoïde (Calder PC., Br J Clin Pharmacol. 2013 Mar;75(3):645-62.), ou des maladies autoimmunes, comme le lupus (Halade GV, Rahman MM, Bhattacharya A, Barnes JL, Chandrasekar B, Fernandes G., J Immunol. 2010 May 1;184(9):5280-6. Epub 2010 Apr 5).

Selon l'invention, le DHA peut être sous forme libre et/ou sous forme de l'un au moins de ses dérivés choisis sous forme d'un glycéride ou d'un phospholipide, sous réserve que ces dérivés soient sous une forme physiologiquement acceptable et possèdent la même activité biologique.

Parmi les dérivés du DHA, sont aussi mentionnés les métabolites et les précurseurs du DHA, les analogues synthétiques ou naturels du DHA ainsi que leurs sels physiologiquement acceptables, et/ou leurs formes estérifiées, et/ou oxygénée (oxylipines, mono-, di-, et tri hydroxy DHA). Les dérivés du DHA selon l'invention c'est-à-dire les dérivés du DHA choisis sous forme d'un glycéride ou d'un phospholipide, sont tous physiologiquement acceptables et possèdent une activité biologique similaire à celle du DHA. Typiquement l'activité biologique d'un dérivé du DHA selon l'invention peut être évaluée comme illustré dans les résultats de la présente demande. En particulier, un dérivé du DHA présente, en association avec la fisétine une activité synergique limitant la différentiation des ostéoclastes en culture (voir figure 1).

Selon l'invention, le DHA pourra être sous forme d'un glycéride ou d'un phospholipide, dans lequel, il pourra ainsi constituer un ou plusieurs des résidus d'acides gras.

Le terme glycéride signifie une molécule de glycérol (i.e., OHCH2CHOHCH2OH) dans laquelle au moins un des trois groupements hydroxyles a été estérifié avec l'acide docosahexaénoïque. L'invention comprend le DHA et ses dérivés sous forme de mono-, di-, ou triglycérides, dans lesquels respectivement un, deux ou les trois groupements hydroxyles ont été estérifiés avec l'acide docosahexaénoïque. Pour les di-ou triglycérides, les groupements hydroxyles sont estérifiés avec un acide gras oméga-3 polyinsaturé (identique ou non), dont au moins un est l'acide docosahexaénoïque (DHA).

A titre d'exemple les composés suivants peuvent être utilisés dans l'invention : Phosphoatidylcholine-DHA, Lyso Phosphoatidylcholine-DHA, Phosphatidylsérine-DHA, etc (et de façon générale tout dérivé du DHA sous forme de phospholipide ou de glycéride). Tout métabolite plus ou moins oxydé, comme RvD1 (7*S*,8*R*,17*S*-trihydroxydocosa-4*Z*,9*E*,11*E*,13*Z*,15*E*,19*Z-*hexaenoic acid), PD1 (10*R*,17*S*-dihydroxydocosa-4*Z*,7*Z*,11*E*,13*E*,15*Z*,19*Z*-hexaenoic acid), 17-HpDHA (17-hydroperoxydocosa-4*Z*,7*Z*,10*Z*,13*Z*,15*E*,19*Z*-hexaenoic acid), 17-HDHA (17-hydroxydocosa-4*Z*,7*Z*,10*Z*,13*Z*,15*E*,19*Z*-hexaenoic acid) est aussi mentionné.

Le DHA et ses dérivés selon l'invention peuvent être obtenus par synthèse chimique, ou purifiés à partir de produits naturels. Selon les modes de réalisation de l'invention, le DHA et/ou ses dérivés peuvent être sous forme plus ou moins purifiée, notamment sous forme d'un ou plusieurs extraits d'un ou plusieurs produits naturels en contenant.

De façon non limitative le DHA peut être obtenu à partir de poissons gras comme le saumon, la truite, le thon, la sardine, le hareng, le maquereau, l'anchois, ou le flétan ; de farines de poisson ; de mollusques ; de fruits de mer ; de crevettes ; de pétoncles ; d'huile de poisson et notamment d'huile d'alose, de foie de morue, de hareng, de maquereau, de sardine ou de saumon ; de tofu ; d'algues ; d'œufs ; d'abats, ou de placenta ; de poulet ; de courge ; de brocoli ; de légumes feuillus, comme la salade, la mâche ou les épinards ; de germe de blé ; de légumes secs, comme les lentilles, les pois ou les haricots ; d'huiles végétales, comme l'huile de colza, de maïs, de chanvre, de tournesol, de canola, de graine de lin, de lin ou de germes de blé ; de graines, comme les graines de courge, de lin ou de chanvre ; de soja ; de chia ; de perilla ; de pourpier ; d'airelles ; de microalgues ; d'algues brunes ; de fruit d'acai ; d'argousier ; de noix.

Dans certains modes de réalisation, le DHA est sous forme d'un extrait de l'un au moins des produits naturels cités ci-dessus.

Le terme sel physiologiquement acceptable inclut les sels des composés d'intérêt de l'invention, préparés à partir d'acides ou de bases non toxiques, en fonction du substituant sélectionné sur le composé d'intérêt.

Des exemples d'acides d'addition physiologiquement acceptables comprennent l'acide hydrochlorique, l'acide méthanesulfonique, l'acide fumarique, l'acide maléïque, l'acide succinique l'acide acétique, l'acide benzoïque, l'acide oxalique, l'acide citrique, l'acide tartrique, l'acide carbonique, l'acide phosphorique. Des exemples de sels d'addition physiologiquement acceptables comprennent l'acétate, le benzènesulfonate, le bicarbonate, le bitartrate, le bromide, le calcium édédate, le calsylate, el carbonate, le chloride, le citrate, le dihydrochloride, l'édétate, l'édisylate, l'estolate, lésylate, le fumarate, le gluceptate, le gluconate, le glutamate, le glycolyllarlanilate, l'héxylresorcinate, l'hydrabamine, l'hydrobromide, l'hydrochloride, l'hydroxynaphtoate, l'iodide, l'isothionate, le lactate, le lactobionate, le malate, le maléate, le mandélate, le mésylate, le méthylbromide, le méthylnitrate, le méthylsulfate, le mucate, le napsylate, le mitrate, le pamoate, le pantothénate, le phosphate, le diphosphate, le polygalacturonate, le salicylate, le stéarate, le subacétate, le succinate, le sulfate le tannate, le tartrate, le téoclate et le trithiodite.

Par excipient physiologiquement acceptable, on entend toute substance, autre que les principes actifs, dans une composition pharmaceutique ou nutritionnelle de l'invention, et non toxique pour l'organisme auquel elle est administrée. Les excipients tels que définis dans la présente demande pourront notamment avoir les caractéristiques suivantes : stabiliser le ou les principes actifs (les liants, les diluants, les conservateurs), solubiliser le ou les principes actifs, permettre une dissolution correcte et ciblée du ou des principes actifs (les délitants), donner une forme (gélule, gel, gouttes, liquide, etc.) ou fournir un support (par exemple solide ou liquide) en rapport avec le mode d'administration (orale, injectable (SC, IV, IM), transcutané, etc.), donner une sapidité nécessaire (par exemple les édulcorants), modifier la biodisponibilité, la demi-vie, etc.

A titre d'exemple on peut citer les excipients suivants : additifs, poudres, colorants, agents de charge, conservateurs, exhausteurs de goût, régulateurs de pH, antioxydants, agents de texture, agent d'enrobage, supports, etc).

De façon générale, les excipients selon l'invention sont adaptés à une administration par voie orale. Dans certains modes de réalisation le ou les principes actifs (i) ou (i) et (ii) de l'invention sont dans un ou des supports adapté(s) à l'administration par voie orale. Préférentiellement ledit support est un support alimentaire, comme décrit ci-après, dans le cadre des compositions nutritionnelles.

Le terme « hydrate » se rapporte aux composés formés par l'addition d'eau ou de ses éléments à une molécule donnée (e.g.: la forme libre du composé) incluant les monohydrates, les dihydrates, etc.

Le terme « solvate » se rapporte aux solvates physiologiquement acceptables, la solvatation étant comprise comme l'interaction d'un soluté avec un solvant permettant la stabilisation dudit soluté dans la solution, et dans laquelle l'état solvaté correspond à une dispersion dans la solution des atomes, ions ou molécules du soluté et à leur interaction avec les molécules de solvant.

Par métabolites, les composés stables issus de la transformation biochimique de la molécule d'intérêt par le métabolisme.

Par précurseur, il est entendu, les composés pharmacologiquement inertes, qui sont rapidement convertis de façon chimique ou enzymatique en une forme bioactive du composé d'intérêt dans un milieu physiologique. Typiquement les précurseurs sont des composés inactifs susceptibles de donner le composé actif d'intérêt une fois dans l'organisme cible.

Le terme produit naturel s'entend dans la présente invention, des produits qui existent, ou qui dérivent, de végétaux, algues, animaux, micro-organismes, à la différence des produits ou composés artificiels (ou de synthèse), obtenus par synthèse chimique notamment.

Selon l'invention, la fisétine, ainsi que le DHA et/ou l'un de ses dérivés peuvent être sous forme d'un extrait de produit naturel en contenant, et notamment des produits d'origine végétale ou animale. Lorsqu'il s'agit d'un extrait végétal (et/ou d'algue), un tel extrait végétal est typiquement obtenu par extraction solide/liquide, éventuellement suivie d'étapes de purification. L'extraction solide-liquide se définit comme une opération de séparation de constituants contenus dans un corps solide par solubilisation avec un solvant par exemple aqueux ou alcoolique. Par extrait végétal il est également compris selon l'invention les plantes (en totalité ou en parties) séchées ou déshydratées et broyées ou réduites en poudre. De tels extraits peuvent être obtenus par pressage, avec ou sans extraction par solvant, expression, hydrodistillation, extraction aqueuse ou alcoolique, filtration fermentation, macération. Cette liste n'étant en aucun cas limitative. Les extraits selon l'invention peuvent être sous forme de jus, concentré aromatique, huile, tisane, infusion, macérât, extrait sec, mou, extrait hydro alcoolique, ou de lyophilisat. De même cette liste n'est également en aucun cas limitative.

Le terme quantité efficace, ou quantité thérapeutiquement efficace, se réfère à la quantité qui est suffisante pour exercer l'activité nutritionnelle et/ou thérapeutique associée, lorsqu'elle est administrée à un sujet. Une telle quantité dépend du sujet, du trouble ou de la pathologie à traiter et/ou à prévenir et du mode d'administration. Elle peut être déterminée par des opérations de routine par l'homme du métier.

Par traitement d'une maladie, d'une pathologie ou d'un trouble, il est entendu, l'inhibition, i.e. l'arrêt du développement, la régression, la disparition des symptômes, des conséquences, et/ou encore des cause de ladite pathologie ou maladie ou dudit trouble.

Par prévention d'une maladie, d'une pathologie ou d'un trouble, il est entendu la prévention de l'apparition de ladite pathologie ou maladie ou dudit trouble, chez un sujet humain ou animal chez qui la maladie de s'est pas encore déclarée.

L'invention est destinée à un usage humain ou vétérinaire, c'est-à-dire qu'elle est destinée à être administrée à l'homme ou à tout autre mammifère parmi lesquels on peut citer par exemple un mammifère domestique comme le chien ou le chat, notamment de grande race ou à pedigree, ou encore les animaux d'élevage tels que le cheval, en particulier un pur-sang et/ou un cheval de course ou les bovins, ovins, caprins ou porcins. Les espèces aviaires qui développent dans certaines conditions des altérations du squelette peuvent également en bénéficier. Par individu, on entend l'homme ou l'animal, et en particulier l'homme.

Par « stimulation de la formation de l'os », on entend, selon l'invention, la capacité de la fisétine, ou de l'un de ses dérivés à stimuler l'activité des ostéoblastes et favoriser ainsi la synthèse de la trame protéique de l'os et le dépôt des minéraux, surtout du calcium, sur cette trame protéique, c'est-à-dire à stimuler la minéralisation de l'os, encore appelée accrétion osseuse.

Par « inhibition de la résorption osseuse », on entend selon l'invention, une inhibition de l'activité de destruction du tissu osseux par les ostéoclastes. Pour vérifier que l'apport de fisétine associé au DHA (et/ou à l'un de ses dérivés), , inhibe la résorption osseuse, l'homme du métier peut mesurer l'activité inhibitrice de la fisétine (et/ou de l'un de ses dérivés), ou de son association avec le DHA (et/ou à l'un de ses dérivés) sur l'inhibition in vitro de la différentiation induite par RANKL, de cellules précurseurs des ostéoclastes (Raw264.7) en cellules multinuclées. Ce test est largement utilisé dans la communauté scientifique (Kawaida R et al., J.Exp. Med., 2003, 197(8) :1029-35 ; Li J et al., PNAS, 2000, 97(4) :1566-71) et est un bon indicateur de l'activité *in vivo* d'un composé, sur la résorption osseuse, comme cela est illustré dans la partie expérimentale.

L'apport de fisétine, associé au DHA et/ou à l'un au moins de ses dérivés, à un organisme animal induit simultanément une stimulation de la formation de l'os et une inhibition de la résorption osseuse (principalement via l'action synergique de l'association fisétine/DHA), ces deux mécanismes permettent *in fine* l'accroissement global de la minéralisation osseuse, et donc de la densité de l'os.

Pour déterminer si un sujet présente un état de masse osseuse réduite, et nécessite en conséquence, un apport de fisétine, associé au DHA et/ou à l'un au moins de ses dérivés, l'homme du métier pourra se référer notamment au rapport de l'Organisation Mondiale de la Santé (OMS) de 1994 intitulé « Assessment of fracture risk and its application to screening for post-menopausal osteoporosis » WHO Technical Series-843).

La fisétine associée au DHA (et/ou à l'un au moins de ses dérivés), ou la composition la (ou les) contenant est destinée à la prévention de la perte osseuse et/ou d'un déséquilibre dans le remodelage du tissu osseux, chez l'homme ou l'animal.

La fisétine associée au DHA (et/ou à l'un au moins de ses dérivés), ou la composition la (ou les) contenant est également destinée à favoriser la croissance osseuse chez les individus jeunes afin d'obtenir des individus possédant une grande densité osseuse et, donc un pic de masse osseuse élevé. En particulier, La fisétine (et/ou de l'un au moins de ses dérivés), éventuellement associé au DHA (et/ou à l'un au moins de ses dérivés), ou la composition la (ou les) contenant est utile pendant la phase de croissance de l'homme ainsi que des autres mammifères.

La fisétine associée au DHA (et/ou à l'un au moins de ses dérivés), ou la composition la (ou les) contenant est aussi destinée aux individus présentant des symptômes de déficit osseux (i.e. : perte osseuse), ou susceptibles de souffrir de déficit osseux, et/ou d'un déséquilibre du rapport entre la formation de l'os et la résorption osseuse lequel, s'il se poursuit, induit une diminution de la masse osseuse.

Une composition selon l'invention est également destinée aux individus présentant des symptômes de déficit osseux résultant d'une fracture, d'une intervention chirurgicale ou encore d'une pathologie dentaire (notamment chez l'enfant par défaut de minéralisation de la dentine ou de l'émail, et chez l'adulte consécutivement à un traitement aux bisphosphonates) (voir Tripathi A et al., J Prosthodont. 2011, 20(7):601-3 et Wittrant Y. et al., Biochim Biophys Acta. 2004 ,1704(2):49-57).

La fisétine associée au DHA (et/ou à l'un au moins de ses dérivés), ou la composition la (ou les) contenant à destination humaine ou vétérinaire, selon l'invention, est en effet utile dans le traitement et/ou la prévention de pathologies, ou de troubles, associés à une perte osseuse, et/ou un déséquilibre entre la formation de l'os et la résorption osseuse, telle que la perte osseuse associée au vieillissement et/ou à la ménopause, la perte osseuse consécutive à une pathologie telle que le diabète ou l'obésité massive, ou due à la prise de certains médicaments tels que notamment les corticoïdes, ou l'ostéolyse observée à proximité d'une prothèse, la maladie de Paget, les maladies tumorales ostéolytiques telles que la majorité des tumeurs osseuses primaires et secondaires (lésions métastatiques de l'os), les maladies ostéolytiques, l'hypercalcémie due à un cancer, les pathologies dentaires et parodontales, et les fractures.

Typiquement la fisétine associée au DHA (et/ou à l'un au moins de ses dérivés), ou la composition la (ou les) contenant est indiquée pour la prévention et/ou le traitement de l'ostéopénie, la raréfaction osseuse, la fragilisation du tissus osseux et /ou l'ostéoporose.

Il a également été montré selon l'invention que l'association de la fisétine et du DHA, selon l'invention, induit une inhibition quasi-totale de la formation des ostéoclastes, sans qu'il soit nécessaire d'ajouter des agents auxiliaires, au contraire de nombreux composés anti-ostéoporose décrits dans l'état de la technique. Cette observation expérimentale souligne l'intérêt de l'association Fisétine /DHA pour inhiber la résorption osseuse, puisque ces composés sont biologiquement actifs utilisés seuls et ne nécessitent pas de co-facteur d'activité.

De façon générale enfin, la fisétine, associée au DHA, ou à l'un de ses dérivés peut être utilisée en prévention de pathologies osseuses pour son effet ostéoprotecteur.

### Mode d'administration d'une composition selon l'invention :

Selon l'invention, l'association comprenant au moins, en tant que principes actifs, (i) la fisétine, et (ii) le DHA, et/ou l'un de dérivés choisis sous forme d'un glycéride ou d'un phospholipide, peut être sous forme d'au moins une composition comprenant en outre au moins un excipient physiologiquement acceptable. Une telle composition est susceptible de prendre plusieurs formes. De façon générale, il est prévu:
(1) une composition comprenant au moins en tant que principes actifs (i) une quantité efficace de fisétine et (ii) une quantité efficace de DHA, et/ou l'un au moins de ses dérivés.
   Une telle composition est également appelée composition (ou préparation) unique et comprend généralement au moins un excipient physiologiquement acceptable. Dans certains modes de réalisation, une composition unique selon l'invention comprend uniquement en tant qu'ingrédients actifs (i) une quantité efficace de fisétine (ii) une quantité efficace de DHA, et/ou l'un au moins de ses dérivés. De façon préférée, lesdits ingrédients actifs sont la fisétine et le DHA.
(2) l'association, ou la composition la contenant, peut également être sous forme d'une trousse, ou d'un produit de combinaison comprenant au moins:
   (i) au moins une quantité efficace de fisétine ; et
   (ii) au moins une quantité efficace de DHA et/ou l'un de ses dérivés;
   pour une utilisation séquentielle, séparée ou simultanée dans le temps.
Les ingrédients actifs (i) et (ii) sont chacun fournis sous une forme qui convient à une administration conjointe de l'un avec l'autre.

L'expression trousse, ou produit de combinaison est utilisée ici en tant que synonyme du terme coffret ou kit et correspond à un ensemble de produits présentés conjointement.

De façon préférée, les ingrédients actifs (i) et (ii) sont respectivement sous forme de compositions (a) et (b) les contenant au moins ; l'une au moins des compositions a) ou b) comprenant en outre au moins un excipient physiologiquement acceptable.

Dans un mode de réalisation particulier d'un produit de combinaison selon l'invention, les ingrédients (i) et (ii) tels que définis ci-dessus sont les seuls principes actifs du produits de combinaison. Plus particulièrement encore, les ingrédients (i) et (ii) consistent en la fisétine et le DHA respectivement.

Selon un autre aspect de l'invention, il est prévu une méthode de fabrication d'une trousse, telle que défini précédemment, ladite méthode comprenant une étape de conditionnement de l'ingrédient actif (i) et de l'ingrédient actif (ii), rendant ainsi les deux ingrédients actifs adaptés à une administration conjointe de l'un avec l'autre.

Les compositions (a) et (b) telles que précédemment définies peuvent être:
(i) sous forme de compositions séparées (i.e. : indépendantes l'une de l'autre), ultérieurement associées pour une utilisation en association l'une avec l'autre, dans le cadre d'un traitement de combinaison.
(ii) présentées ensemble dans un même contenant sous forme de compositions séparées dans un conditionnement combiné pour une utilisation en association l'une avec l'autre, dans le cadre d'un traitement de combinaison.

Une trousse selon l'invention pourra également contenir des instructions pour l'utilisation des composants qu'elle contient.

Dans le cadre des produits de combinaison sous forme de trousse, l'expression «administration conjointe», selon la présente invention, inclut que les ingrédients actifs (i) et (ii) ou les compositions (a) et (b) comprenant respectivement au moins (i) la fisétine, et au moins un excipient physiologiquement acceptable ; et (ii) au moins le DHA ou l'un de ses dérivés, et au moins un excipient physiologiquement acceptable, sont administrés séquentiellement (i.e. : successivement, de façon étalée dans le temps), séparément et/ou simultanément pendant la période de traitement.

Conformément au produit de combinaison selon l'invention, le terme «administration conjointe», inclut que les ingrédients actifs (i) et (ii) du produit de combinaison sont administrés (éventuellement de façon répétée) soit ensembles, soit séparément, de façon suffisamment rapprochée dans le temps, pour permettre un effet bénéfique synergique entre les deux composants, tel que défini précédemment. La détermination d'un tel effet synergique dépendra notamment de l'effet recherché, et/ou du patient, et/ou de la formulation des composants.

Les compositions selon l'invention peuvent ainsi être administrées de façon simultanées, sous forme d'une composition unique ou bien sous forme de compositions distinctes. Lorsque les compositions sont distinctes elles peuvent également être administrées de façon séquentielle, par exemple à des moments différents de la journée, (ainsi une composition comprenant au moins l'un des ingrédients actifs (i) ou (ii) pourrait être administrée le matin et l'autre pourrait être administrée le midi) et/ou à des fréquences quotidiennes.

L'expression « en association l'une avec l'autre » inclut que l'une ou l'autre des deux compositions (a) ou (b) peut être administrée (éventuellement de façon répétée voire continue) avant, après, et/ou en même temps que l'administration de l'autre composition.

Il est par exemple envisageable que chacune des compositions (a) et (b) soient administrées à des moments différents de la journée, ou que l'une des deux compositions (a) ou (b) soit prise à une fréquence différente de l'autre. Les deux compositions peuvent être administrées par voie entérale, en particulier par voie orale, et/ou par voie parentérale (intramusculaire, intraveineuse, intradermique, sous-cutanée) selon des fréquences différentes ou identiques. Une composition pouvant être prise par bolus quotidiennement, par exemple le matin ou le soir, et l'autre composition de façon fractionnée en plusieurs prises quotidiennes, par exemple, matin et soir ou encore, matin, midi et soir.

Lorsque les ingrédients actifs (i) et (ii) sont présents dans deux compositions séparées (a) et (b), il est également possible de prendre les dits ingrédients selon des durées différentes ou encore de les prendre séquentiellement (une cure après l'autre ou simplement à des moments différents de la journée). A titre d'exemple, une composition (a) comprenant au moins (i) la fisétine pourrait être administrée quotidiennement, à raison d'une à trois fois par jour, et une composition (b) comprenant au moins le DHA ou l'un de ses dérivés, pourrait être prise quotidiennement une fois par jour.

L'administration d'un produit de combinaison tel que décrit dans la présente invention pourra donc être réalisée de façon répétée, sous forme de cure, pendant plusieurs jours ou plusieurs semaines (par exemple 4 semaines). Eventuellement, plusieurs cures successives peuvent être entreprises par exemple 2 à 4, éventuellement interrompues par une pause de 1 semaine à 3 mois.

Dans le cadre d'un traitement préventif, l'administration d'un produit de combinaison selon l'invention est typiquement réalisée de façon chronique.

Lorsque le produit de combinaison se présente sous forme d'une trousse contenant au moins une composition (a) et une composition (b) distinctes, telles que précédemment décrites, il est entendu que les deux compositions peuvent être dans des formulations identiques ou similaires et administrées suivant des voies entérales ou parentérales identiques ou similaire. De façon préférée, les compositions de l'invention sont sous forme adaptée à la voie orale, néanmoins, les compositions peuvent être indépendamment l'une de l'autre sous l'une quelconque des formes solides ou liquides décrites ci-après.

Dans certains modes de réalisation de l'invention, la fisétine (i), est administrée à une dose allant de 0.01 à 1 g/jour, de préférence allant de 0.1 à 1 g/jour et le DHA (ii), ou l'un de ses dérivés, est administré à une dose allant de 0.01 à 3 g/jour, de préférence allant de 0.1 à 1 g/jour. Une telle dose quotidienne peut être administrée en une ou plusieurs prises.

Une composition selon l'invention, comprenant au moins en tant que principes actifs (i) la fisétine, associée au (ii) DHA, et/ou à l'un au moins de ses dérivés, peut se trouver sous la forme d'une composition nutritionnelle ou d'une composition pharmaceutique, telles que décrites ci-après.

Préférentiellement ladite composition comprend (i) la fisétine, et (ii) le DHA, et/ou l'un au moins de ses dérivés, et au moins un excipient physiologiquement acceptable. Suivant les modes de réalisations de l'invention, la fisétine, et le DHA, et/ou l'un au moins de ses dérivés peuvent être les seuls principes actifs ou associés à d'autres principes actifs ayant ou non la même indication thérapeutique ou nutritionnelle.

La formulation des produits de combinaison selon l'invention ainsi que la nature et la quantité des ingrédients actifs dans la composition unique ou les compositions de la trousse selon l'invention dépendent de la finalité thérapeutique ou nutritionnelle du traitement.

### Compositions nutritionnelles :

L'invention a également pour objet une composition nutritionnelle pour son utilisation pour prévenir et/ou traiter les pathologies nécessitant de stimuler la formation de l'os et/ou d'inhiber la résorption osseuse, caractérisée en ce qu'elle comprend, à titre de composé(s) nutritionnel(s) actif(s), (i) la fisétine associée (ii) au DHA, ou à l'un de ses dérivés choisis sous forme d'un glycéride ou d'un phospholipide.

Une telle composition nutritionnelle peut être plus particulièrement destinée à :
- stimuler la formation de l'os chez les individus jeunes en phase de croissance, à prévenir la perte osseuse qui se produit avec le vieillissement, et/ou avec la ménopause, et/ou consécutivement à une pathologie telle que le diabète ou l'obésité massive et/ou à la prise de certains médicaments tels que les corticoïdes ;
- prévenir ou traiter des troubles liés à un déséquilibre du rapport entre la formation de l'os et la résorption osseuse ;
- traiter un déficit osseux résultant d'une fracture ;
- prévenir ou traiter des pathologies choisies parmi , la maladie de Paget, la perte osseuse, les pathologies ou troubles associés à une perte osseuse, comme les maladies dentaires et parodontales, les maladies, troubles ou affections ostéolytiques, comme l'ostéolyse observée à proximité d'une prothèse et les maladies tumorales ostéolytiques, telles que la majorité des tumeurs osseuses primaires et secondaires (lésions métastatiques de l'os), et l'hypercalcémie due à un cancer;
- prévenir ou traiter, l'ostéopénie, la raréfaction osseuse, la fragilisation du tissu osseux, l'ostéoporose.

Par « composition nutritionnelle », on entend selon l'invention, une composition telle que décrite précédemment, et constituant une composition alimentaire ou encore un supplément alimentaire ne possédant pas les caractéristiques d'un médicament.

Une composition nutritionnelle selon l'invention est préférentiellement adaptée pour une administration orale.

Une composition nutritionnelle selon l'invention peut être un aliment diététique utilisé pour le maintien d'une bonne santé de l'homme ou de l'animal qui l'ingère. Une telle composition alimentaire est également communément désignée « aliment fonctionnel », et est destinée à être consommée, soit comme partie intégrante du régime alimentaire, soit comme supplément (ou complément) alimentaire, mais dont le contenu en principe actif ((i) la fisétine, associée avec (ii) le DHA, et/ou l'un au moins de ses dérivés) implique un rôle physiologique allant au-delà de la fourniture des besoins nutritifs de base.

Une composition nutritionnelle selon l'invention, caractérisée en ce qu'elle comprend (i) la fisétine, associée avec (ii) le DHA (et/ou l'un au moins de ses dérivés), en tant que composés actifs, peut se présenter sous une grande diversité de formes de compositions alimentaires et de boissons, y compris sous forme de produits laitiers, comme le lait, et notamment des laits infantiles et/ou de croissance, des yaourts, des desserts lactés, des glaces, des fromages ; des dérivés de laits végétaux ou de fruits, légumes ou algues, comme des jus, préférentiellement des jus de fruits, des salades de fruits, des compotes de fruits, des potages; des produits céréaliers, comme le pain, les biscuits et gâteaux, des barres de céréales, des céréales pour le petit déjeuner ainsi que des céréales infantiles, des dérivés de produits issus de la mer, notamment les produits dérivés de poissons, des corps gras tels des huiles ou des substituts de beurre, les pâtes à tartiner, ou encore des plats cuisinés. Une composition pourra encore être sous forme de desserts, des produits de confiserie, de produits d'assaisonnement des aliments (notamment épices et sauces), de confiseries, etc.

Une composition nutritionnelle selon l'invention, caractérisée en ce qu'elle comprend (i) la fisétine associée avec (ii) le DHA (et/ou l'un au moins de ses dérivés), en tant que composés actifs, peut aussi se présenter sous la forme d'une grande diversité de produits destinés à l'alimentation animale, en particulier pour le chien ou le chat, ou en encore le cheval ou les animaux d'élevage, qu'ils soient sous forme humide, sous forme semi-humide ou sous forme sèche , notamment sous la forme de croquettes.

La fisétine et le DHA, ainsi que les dérivés du DHA selon l'invention peuvent se trouver sous forme plus ou moins purifiée. Ils peuvent être obtenus par synthèse chimique ou purifiés à partir de produits naturels en contenant. Dans certains modes de réalisation, la fisétine et/ou le DHA (ou l'un de ses dérivés) sont des molécules de synthèse ; dans d'autres modes de réalisation la fisétine et/ou le DHA (ou l'un de ses dérivés) sont sous forme d'extraits naturels en contenant.

En particulier, comme cela a été décrit précédemment, la fisétine ou l'un de ses dérivés peut se trouver sous forme d'extrait de fraise, de mangue, de kiwi, de pêche, de kaki, de pomme, de tomate, d'oignons, de concombre, de certains arbres du genre Acacia, (*Acacia greggii, Acacia berlandieri*), de l'arbre Quebracho colorado, du cyprès de Nootka (*Xanthocyparis nootkatensis*), de fleurs *Butea monosperma* (palash, flamme de la forêt), et/ou de plantes du genre Rhus. Dans certains modes de réalisation on utilisera des extraits de fraise, de mangue, de certains arbres du genre Acacia, (*Acacia greggii, Acacia berlandieri*), de l'arbre Quebracho colorado, du cyprès de Nootka (*Xanthocyparis nootkatensis*), de fleurs *Butea monosperma* (palash, flamme de la forêt), et/ou de plantes du genre *Rhus.*

Le DHA ou ses dérivés selon l'invention peuvent se trouver sous forme d'extraits de poissons gras comme le saumon, la truite, le thon, la sardine, le hareng, le maquereau, l'anchois, ou le flétan ; de farines de poisson ; de mollusques ; de fruits de mer ; de crevettes ; de pétoncles ; d'huile de poisson et notamment d'huile d'alose, de foie de morue, de hareng, de maquereau, de sardine ou de saumon ; de tofu ; d'algues ; d'œufs ; d'abats, ou de placenta ; de poulet ; de courge ; de brocoli ; de légumes feuillus, comme la salade, la mâche ou les épinards ; de germe de blé ; de légumes secs, comme les lentilles, les pois ou les haricots ; d'huiles végétales, comme l'huile de colza, de maïs, de chanvre, de tournesol, de canola, de graine de lin, de lin ou de germes de blé ; de graines, comme les graines de courge, de lin ou de chanvre ; de soja ; de chia ; de perilla ; de pourpier ; de airelles ; de microalgues ; d'algues brunes ; de fruit d'acai ; d'argousier et/ou de noix.

Une composition nutritionnelle selon l'invention, qui se présente sous une forme liquide ou sous une forme solide, notamment sous la forme d'une poudre, gélule, lyophilisat, granule, comprimé, capsule, sirop, infusion, jus ou macérât, peut donc consister en un produit d'extraction obtenu à partir d'au moins un composé naturel tel que décrit précédemment comprenant (i) la fisétine et au moins un composé naturel comprenant (ii) le DHA (et/ou l'un de ses dérivés). Une composition nutritionnelle selon l'invention peut également comprendre un produit d'extraction obtenu à partir desdits composés naturels.

A titre d'exemple une composition nutritionnelle selon l'invention peut se présenter sous la forme d'un jus, d'une infusion ou d'un macérât de fruits et/ou de légumes, en particulier un jus de fraise, de mangue, de kiwi, de pêche, de kaki, de pomme, de tomate, d'oignons, et/ou de concombre, de préférence associé avec du jus d'airelles ; de fruit d'acai , de courge ; de brocoli; de légumes feuillus, comme la salade, la mâche ou les épinards et/ou d'argousier.

Une composition nutritionnelle selon l'invention peut également se présenter sous la forme d'une boisson, par exemple de l'eau minérale, à laquelle a été ajoutée un extrait d'un composé naturel comprenant (i) la fisétine et à partir d'au moins un composé naturel comprenant (ii) le DHA et/ou au moins l'un de ses dérivés.

L'utilisation de principes actifs (i) et (ii) d'origine naturelle, selon l'invention est extrêmement avantageuse car les produits naturels contenant ces principes actifs, comme les fruits et notamment, les fraises, pommes, mangues pour la fisétine et le poisson et ses dérivés (farines, huile), légumes secs ou noix pour le DHA sont associés à une image d'une grande naturalité, c'est-à-dire des produits alimentaires sains, également vecteurs d'une notion de plaisir. Un tel traitement nutritionnel peut donc être aisément inclus dans le cadre d'une alimentation équilibrée pour une durée très longue, caractéristique des affections chroniques. Par ailleurs, ces composés peuvent être pris directement dans le cadre d'un traitement nutritionnel, sans transformation et/ou ajout de substances complémentaires.

En outre, ces composés naturels, sont facilement disponibles (ils peuvent être produits à grande échelle dans un contexte régional et/ou Européen) et peuvent être stockés aisément.

Dans un mode de réalisation particulier, la composition nutritionnelle de l'invention comprend en tant que composés nutritionnels actifs (i) et (ii), la fisétine et la DHA respectivement. Plus particulièrement encore, ladite composition comprend la fisétine et le DHA en tant que seuls composés nutritionnels actifs. Ces composés nutritionnels actifs (i) et (ii) peuvent être sous forme plus ou moins purifiée, notamment sous forme d'extraits naturels comme décrit plus haut ou peuvent être produits par synthèse chimique. Plus particulièrement encore, ladite composition comprend la fisétine et le DHA en tant que seuls composés nutritionnels actifs.

Une composition nutritionnelle selon l'invention pourra être sous la forme d'une composition unique ou d'un produit de combinaison, tel que précédemment décrits.

De préférence, une composition nutritionnelle, adaptée à un usage humain ou vétérinaire selon l'invention, comprend une quantité de fisétine adaptée à une administration orale quotidienne allant de 0,01 à 1 g, de préférence allant de 0,1 à 1 et éventuellement une quantité de DHA, et/ou de l'un de ses dérivés allant de 0,01 à 3 g, de préférence, de 0,1 à 1 g.

Dans certains modes de réalisation, la fisétine, peut être présente dans une composition de l'invention à une teneur (en poids par rapport au poids total de la composition) allant de 1 à 98.9 %, de préférence à une teneur allant de 1 à 80 %, de 1 à 50 %, de 1 à 20 %, de 1 à 10 %, de 1 à 5 %, de 1 à 2 % ; de même le DHA, ou l'un de ses dérivés, peut être présent à une teneur allant de 1 à 98.9 %, de 1 à 80%, de préférence à une teneur allant de 1 à 50 %, de 1 à 20 %, de 1 à 10 %, de 1 à 5 %, de 1 à 2 %.

De préférence, une composition nutritionnelle, adaptée à un usage humain ou vétérinaire selon l'invention, comprendra en tant que principes actifs au moins (i) la fisétine et (ii) le DHA et/ou l'un de ses dérivés. De préférence encore, une telle composition contient un ratio Fisétine/DHA allant variant de 100/1 à 1/100, en particulier de 50/1 à 1/50, de 20/1 à 1/50, de 10/1 à 1/50, de 10/1 à 1/10, de 1/1 à 1/50, ou de 1/1 à 1/10.

Une composition nutritionnelle selon l'invention comprend, outre les principes actifs, au moins un excipient ou support physiologiquement acceptable. De tels excipients, ou supports physiologiquement acceptables ont été décrits précédemment. De façon préférée, ceux-ci sont adaptés à une administration par voie orale. Dans certains modes de réalisation d'une composition nutritionnelle selon l'invention, un support adapté peut être un support alimentaire, notamment lorsque les compositions sont sous forme de composition alimentaire.

Une composition nutritionnelle selon l'invention pourra être sous la forme d'une composition unique ou d'un produit de combinaison, tel que précédemment décrits.

Dans un mode de réalisation, la composition nutritionnelle de l'invention comprend en tant que composés nutritionnels actifs (i) et (ii), la fisétine et la DHA respectivement.

Selon un mode de réalisation particulier, ladite composition comprend la fisétine et le DHA en tant que seuls composés nutritionnels actifs. Ces composés nutritionnels actifs (i) et (ii) peuvent être sous forme plus ou moins purifiée, notamment sous forme d'extrait naturels comme décrit plus haut ou peuvent être produits par synthèse chimique. Plus particulièrement encore, ladite composition comprend la fisétine et le DHA en tant que seuls composés nutritionnels actifs.

Dans un autre mode de réalisation, une composition nutritionnelle selon l'invention peut comprendre d'autres composés, en combinaison avec (i) la fisétine et (ii) le DHA (et/ou l'un de ses dérivés). De tels composés additionnels viseront par exemple à rétablir l'équilibre entre formation et résorption osseuse, et/ou à préserver ou améliorer la santé osseuse, et/ou permettre une meilleure assimilation par l'organisme des principes actifs de la composition, améliorer le goût ou la consistance de la composition.

A titre d'exemple, une composition nutritionnelle de l'invention peut comprendre de façon non-limitative l'un au moins des composés mentionnés ci-après.

La composition nutritionnelle selon l'invention peut comprendre une source de calcium, par exemple sous la forme d'un composé organique ou inorganique physiologiquement acceptable, tels que des sels de calcium inorganique (chlorure de calcium, phosphate de calcium, sulfate de calcium, oxyde de calcium, hydroxyde de calcium ou carbonate de calcium) ou des composants organiques contenant du calcium telle que la poudre de lait écrémé, le caséinate de calcium ou encore des sels organiques de calcium (citrate de calcium, maléate de calcium ou leurs mélanges).

La quantité de calcium contenue dans une composition nutritionnelle selon l'invention est adaptée pour une administration quotidienne, fournie par ladite composition, comprise entre 100 mg et 1000 mg, de préférence entre 200 mg et 700 mg et de manière tout à fait préférée entre 300 mg et 600 mg de calcium.

Une composition nutritionnelle selon l'invention peut également comprendre des vitamines, telle que la vitamine A, la vitamine D, la vitamine E, la vitamine K, la vitamine C, l'acide folique, la thiamine, la riboflavine, la vitamine B6, la vitamine B12, la niacine, la biotine ou encore l'acide pantothénique.

Une composition nutritionnelle selon l'invention peut également comprendre des éléments minéraux et des éléments trace tels que le sodium, le potassium, le phosphore, le magnésium, le cuivre, le zinc, le fer, le sélénium, le chrome et le molybdène.

Elle peut également comprendre des fibres solubles telles que l'agar-agar, un alginate, du caroube, du carragheenan, de la gomme arabique, de la gomme de guar, de la gomme de karaya, de la pectine ou de la gomme xanthane, ces fibres solubles étant sous une forme hydrolysée ou non hydrolysée.

Elle peut aussi comprendre des composés source d'énergie, par exemple une ou plusieurs sources d'hydrates de carbone choisis parmi les maltodextrines, l'amidon, le lactose, le glucose, le sucrose, le fructose, le xylitol et le sorbitol, ou encore des protéines, peptides ou acides aminés.

Elle peut aussi comprendre d'autres types de substances comme par exemple des fibres alimentaires, notamment des fibres insolubles et/ou des prébiotiques.

En outre, une composition nutritionnelle selon l'invention peut également comprendre des arômes naturels ou artificiels, par exemple des arômes de fruits comme la banane, l'orange, la pêche, l'ananas ou la framboise, ou d'autres arômes végétaux comme la vanille, le cacao, le café, etc..

Dans certains modes de réalisation de l'invention, l'association, ou la composition la contenant, comprend en outre, en tant que principe actif l'acide eicosapentaénoïque (EPA) ou l'un de ses dérivés c'est à dire les métabolites et les précurseurs de l'EPA, ainsi que ces sels physiologiquement acceptables, et/ou ces formes estérifiées, et/ou oxygénée (oxylipines, mono-, di-, et tri hydroxy DHA). Par dérivés de l'EPA on entend également les formes glycérides ou phospholipides. Selon ce mode de réalisation, l'EPA, ou l'un de ses dérivés, pourra être obtenu par synthèse chimique, ou purifié à partir d'un composé naturel. L'EPA pourra donc être sous forme plus ou moins purifiée, notamment sous forme d'extrait naturel en contenant.

Compositions pharmaceutiques humaines ou vétérinaires selon l'invention.

L'invention a également pour objet une composition pharmaceutique humaine ou vétérinaire pour le traitement de pathologies nécessitant une simulation de la formation de l'os, caractérisée en ce qu'elle comprend, à titre de principe actif, au moins (i) la fisétine et (ii) le DHA.

De préférence, une telle composition pharmaceutique selon l'invention comprend au moins à titre de principes actifs, (i) la fisétine (ii) le DHA et/ou l'un au moins de ses dérivés, et sera ainsi particulièrement indiquée pour inhiber la résorption osseuse chez l'homme ou l'animal.

Typiquement, une composition comprenant au moins à titre de principes actifs, (i) la fisétine et (ii) le DHA et/ou l'un au moins de ses dérivés est utilisée pour la prévention/ou le traitement de pathologies nécessitant une inhibition de la résorption osseuse, et/ou la prévention ou le traitement d'une pathologie liée à un déséquilibre du métabolisme osseux, c'est-à-dire une composition pharmaceutique apte à inhiber la résorption osseuse.

Une composition pharmaceutique selon l'invention comprend au moins, en tant que principe actif, la fisétine et le DHA en une quantité adaptée pour stimuler la formation de l'os.

De façon préférée, une composition pharmaceutique selon l'invention comprend, en tant que principe actif, la fisétine et le DHA, et/ou l'un de ses dérivés en quantités suffisante pour permettre un effet synergique sur l'inhibition de la différentiation des ostéoclastes et par conséquent sur l'inhibition de la résorption osseuse.

Une composition pharmaceutique selon l'invention est également utile pour stimuler la formation de l'os chez les individus jeunes, (homme ou animaux en phase de croissance), afin d'augmenter la densité osseuse atteinte au début de l'âge adulte et accroître la masse osseuse maximale (pic de masse osseuse) au début de l'âge adulte ; prévenir la perte osseuse associée au vieillissement et/ou à certaines pathologies, et/ou à la prise de médicaments; traiter un déficit osseux résultant d'une fracture, prévenir ou traiter des troubles ou des pathologies associés à une perte osseuse et/ou à déséquilibre du remodelage osseux, telles que l'ostéoporose, la maladie de Paget, les maladies dentaires et parodontales, l'hypercalcémie due à un cancer, les maladies, troubles ou affections ostéolytiques comme la perte osseuse ou l'ostéolyse observée à proximité d'une prothèse et les maladies tumorales ostéolytiques telles que la majorité des tumeurs osseuses primaires et secondaires (lésions métastatiques de l'os), ; traiter ou prévenir l'ostéopénie, la fragilisation du tissu osseux et/ou la raréfaction osseuse.

Il peut s'agir d'une composition pharmaceutique humaine ou vétérinaire. Une composition pharmaceutique selon l'invention comprend au moins la fisétine et le DHA en association avec au moins un excipient choisi dans le groupe constitué par les excipients physiologiquement acceptables. De préférence, une composition pharmaceutique de l'invention comprend en tant que principe actif, (i) la fisétine, et (ii) le DHA ou l'un de ses dérivés, en association avec au moins un excipient physiologiquement acceptable.

Dans un mode de réalisation, les principes actifs (i) et (ii) d'une telle composition sont respectivement la fisétine et le DHA.

Dans un mode de réalisation particulier, les principes actifs d'une telle composition consistent en la fisétine, et le DHA, ou l'un de ses dérivés.

Les principes actifs (i) et (ii) tels que définis précédemment peuvent être sous forme plus ou moins purifiée, notamment obtenus à partir de synthèse chimique ou sous forme d'extraits naturels en contenant. De tels extraits ont été décrits précédemment également.

La composition pharmaceutique selon l'invention se présente sous une forme adaptée pour l'administration orale ou parentérale, notamment par voie intraveineuse (directement ou par perfusion), sous-cutanée, intradermique ou intramusculaire. Les formulations, et notamment les excipients, seront adaptées par l'homme du métier en fonction du mode d'administration choisi.

De préférence, une telle composition se présente sous une forme adaptée à l'administration par voie orale.

Dans ce mode de réalisation, le (ou les) excipient(s), ou le support physiologiquement acceptable(s), sont adaptés à l'administration par voie orale. Des adjuvants, des véhicules et des excipients physiologiquement acceptables sont aussi décrits dans l'ouvrage intitulé "Handbook of Pharmaceutical Excipients, Seconde édition, American Pharmaceutical Association, 1994.

Une composition pharmaceutique selon l'invention se présente indifféremment sous une forme solide ou sous une forme liquide. Différentes formulations ont été précédemment décrites, ainsi que des excipients physiologiquement acceptables.

Pour une administration orale, on préférera une composition pharmaceutique solide, sous la forme de comprimés, de capsules ou de gélules.

Sous la forme liquide, on préférera une composition pharmaceutique sous la forme d'une suspension aqueuse ou d'une suspension non aqueuse, ou encore sous la forme d'une émulsion eau-dans-huile ou huile-dans-eau.

Des formes pharmaceutiques solides peuvent comprendre, en tant que véhicules, adjuvants ou excipients, au moins un agent diluant, un arôme, un agent solubilisant, un agent lubrifiant, un agent de suspension, un agent liant, un agent désintégrant et un agent d'encapsulation. De tels composés sont par exemple le carbonate de magnésium, le stéarate de magnésium, le talc, le lactose, la pectine, la dextrine, l'amidon, la gélatine, des matériaux cellulosiques, du beurre de cacao, etc. Les compositions sous forme liquide peuvent comprendre également de l'eau, le cas échéant en mélange avec du propylène glycol ou du polyéthylène glycol, et éventuellement aussi des agents de coloration, des arômes, des stabilisants et des agents épaississants.

Des techniques de préparation de compositions pharmaceutiques selon l'invention peuvent être aisément retrouvées par l'homme du métier, par exemple dans l'ouvrage Remmingston's Pharmaceutical Sciences, Mid. Publishing Co, Easton, PA, USA.

Pour formuler une composition pharmaceutique selon l'invention, l'homme du métier pourra avantageusement se référer à la dernière édition de la Pharmacopée Européenne ou de la Pharmacopée des Etats-Unis d'Amérique (USP).

L'homme du métier pourra notamment avantageusement se référer à la quatrième édition « 2002 » de la Pharmacopée Européenne, ou encore à l'édition USP 25-NF 20 de la Pharmacopée Américaine (U.S. Pharmacopeia).

Des ratios Fisétine /DHA (ou l'un de ses dérivés) ainsi que des concentrations de ces principes actifs dans des compositions unitaires ont été mentionnés dans le cadre des compositions nutritionnelles et sont également adaptés aux compositions pharmaceutiques selon l'invention.

Comme mentionné précédemment, une composition de l'invention, en particulier une composition pharmaceutique, peut comprendre ou la fisétine et le DHA, ou l'un des dérivés du DHA choisi sous forme d'un glycéride ou d'un phospholipide, des principes actifs supplémentaires. De tels principes actifs pourront avoir une indication identique à la fisétine ou l'association selon l'invention, ou bien avoir une indication thérapeutique différente. Préférentiellement, dans ce mode de réalisation, des principes actifs supplémentaires viseront à prévenir ou traiter les pathologies associées avec un déséquilibre de la formation et de la résorption osseuse et donc à stimuler la formation osseuse et/ou à inhiber la perte osseuse.

Des principes actifs supplémentaires ont notamment été décrits dans la partie relative aux compositions nutritionnelles et sont également adaptées aux compositions pharmaceutiques de l'invention.

L'invention concerne aussi une méthode pour prévenir ou traiter un trouble lié à un déséquilibre du métabolisme osseux, en particulier un trouble associé à une perte de la masse osseuse, ladite méthode comprenant une étape au cours de laquelle on administre aux patients une quantité thérapeutiquement efficace de fisétine associée à une quantité thérapeutiquement efficace de DHA et/ou de l'un de ses dérivés ou encore d'une composition pharmaceutique contenant la fisétine associée au DHA et/ou l'un de ses dérivés.

L'invention est en outre illustrée, sans pour autant être limitée, par les résultats expérimentaux suivants.

### EXEMPLES

### 1- Matériels et Méthode:

### Réactifs :

Les réactifs ont été achetés chez Sigma-Aldrich (St Louis, MO, USA: fisetin - ref F4043, DHA - ref D2534, bêta-glycérophosphate, acide ascorbique, LPS sérotype 026:B6, hydroxypropylcellulose, p-nitrophényl-phosphate, BSA), R&D Systems Europe Ltd (Abingdon, Royaume-Uni: RANKL), Extrasynthèse (Genay, France:. fisetin - ref 1167 S), Invitrogen-Life technologies (Saint-Aubin, France : TRIzol).

### Culture cellulaire

Les préostéoblastes primaires ont été isolés à partir de la calotte crânienne de rats Wistar nouveaux-nés par digestion enzymatique comme précédemment décrit [Declercq, H., Van den Vreken, N., De Maeyer, E., Verbeeck, R., Schacht, E., De Ridder, L., and Cornelissen, M. (2004). Isolation, proliferation and differentiation of osteoblastic cells to study cell/biomaterial interactions: comparison of different isolation techniques and source. Biomaterials 25, 757-68.]. Les précurseurs d'ostéoclastes RAW264.7 ont été obtenus auprès de l'ATCC (Manassas, VA, USA). Les préostéoblastes et les RAW264.7 ont été cultivés dans du milieu α-MEM (Invitrogen, Carlsbad, CA, USA) supplémenté avec 10% de SVF décomplémenté et de 100 unités/ml de pénicilline-streptomycine.

### Coloration TRAP

Pour la coloration TRAP, 60.000 cellules RAW264.7 ont été ensemencées dans des puits de plaques 12 puits pendant 12 heures puis traitées avec du DMSO/éthanol comme témoin ou de la fisétine (5 µM dans le DMSO) ou du DHA (50µM dans l'éthanol) ou la combinaison des deux (Fisétine 5 µM et DHA 50 µM) pendant 3 heures. Les cellules ont ensuite été induites à se différencier en présence de RANKL (50 ng/ml) avec du DMSO/éthanol ou de la fisétine (5 µM) ou du DHA (50 µM) ou la combinaison des deux (Fisétine 5 µM et DHA 50 µM) durant 4 jours avec un changement de milieux après 2 jours. De la BSA délipidée et sans endotoxines a été utilisée pour solubiliser le DHA avec un rapport 1/5 (BSA/DHA).

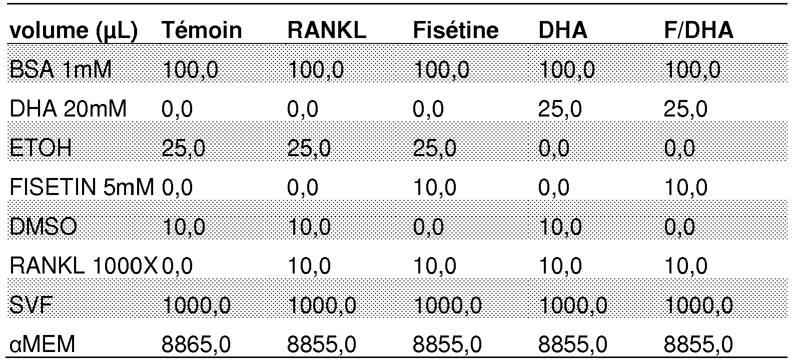

La coloration TRAP a été réalisée en utilisant le kit « leukocyte acid phosphatase » (Sigma-Aldrich) selon les recommandations du fournisseur après fixation des cellules. Les cellules TRAP+ multinucléées ont ensuite été comptées.

### Tests de minéralisation

Les préostéoblastes primaires ont été ensemencés à 1,5 * 10⁵ cellules par puit dans des plaques 12 puits traitées au collagène. Trente-six heures plus tard, les cellules ont été prétraitées avec du DMSO comme témoin ou de la fisétine (1 à 5 µM) pendant 12 heures, puis induites à se différentier avec 25 µg/ml d'acide ascorbique et 5 mM de bêta-glycérophosphate en présence de DMSO ou de fisétine (1 à 5 µM). Après 17 jours de culture avec un changement des milieux tous les 2 jours, les cellules ont été fixées avec de l'éthanol à 70% et les dépôts de calcium ont été colorés avec une solution à 40 mM d'alizarine rouge (pH 4,2). La zone et l'intensité des nodules ont été quantifiées en utilisant le logiciel ImageJ (http://rsb.info.nih.gov/ij/).

### Mesure de l'activité ALP

Les préostéoblastes primaires ont été ensemencés à 3500 cellules par puits dans des plaques 96 puits traitées au collagène. Trente-six heures plus tard, les cellules ont été prétraitées avec du DMSO comme témoin ou de la fisétine (1 à 5 µM) pendant 12 heures, puis induites à se différentier avec 25 µg/ml d'acide ascorbique et 5 mM de bêta-glycérophosphate en présence de DMSO ou de fisétine (1 à 5 µM). L'activité enzymatique ALP a été mesurée en cinétique après 0, 5, 7 et 12 jours de traitement avec un changement de milieux tous les 2 jours. Les cellules ont été lysées par des cycles de gel-dégel et les lysats ont subi une homogénéisation dans 200 µl de tampon chlorure de magnésium hexahydrate, di-éthanoamine à un pH de 9,8. Les lysats cellulaires (10 µl) ont été ajoutés à 200 µl de solution de p-nitrophényl phosphate (16,2 mM) et l'absorbance a été mesurée en utilisant un lecteur de microplaques à 405 nm toutes les 90 seconds pendant 30min. Pour chaque échantillon, la concentration de protéines a été analysée pour exprimer les résultats sous forme de µmol de p-nitrophenol/heure/mg de protéines.

### RT-PCR quantitative

L'extraction des ARN totaux (cellules et fémurs) a été effectuée en utilisant le réactif TRIzol, la transcription inverse (RT) a été réalisée à l'aide du kit de synthèse d'ADNc Dynamo (Thermo Scientific, Waltham, MA, USA) et l'analyse PCR en temps réel a été réalisée avec le Mastercycler ep Realplex2 (Eppendorf, Hambourg, Allemagne). Toutes les valeurs ont été normalisées par la GAPDH. Les séquences des oligos utilisés sont : ALP F 5'-GGCCAGCTACACCACAACA-3' / ALP R 5'-CTGAGCGTTGGTGTTATATGTCTT -3' / OCN F 5'-AGACTCCGGCGCTACCTT -3' / OCN R 5'-CAAGCAGGGTTAAGCTCACA-3'/ COL1 F 5'-CATGTTCAGCTTTGTGGACCT-3' / COL1 R 5'-GCAGCTGACTTCAGGGATGT-3' / GAPDH F 5'-AACTTTGGCATTGTGGAAGG-3' / GAPDH R 5'-GGATGCAGGGATGATGTTCT-3'

### Etudes animales

Tout au long de l'étude, les animaux ont été logés dans un environnement contrôlé (12:12 h cycle lumière-obscurité, 20-22 ° C, 50-60% d'humidité relative, 1 souris par cage en plastique avec un accès libre à l'eau. Les souris C57BL/6J ont été livrées 2 semaines avant l'étude pour l'acclimatation à notre environnement sous régime standard semi-purifié. Pour toutes les expériences, les souris de 8 semaines (n=12/groupe) ont été gavées avec le véhicule (1% d'éthanol, 0,2% hydroxypropylcellulose dans de l'eau distillée) ou avec la fisétine (5 à 50 mg/kg dans le véhicule) pendant une semaine. Ensuite, pour l'expérience OVX, les souris ont été pseudo-opérées ou ovariectomisées bilatéralement et ont reçu quotidiennement par gavage le véhicule ou la fisétine pendant 4 semaines. A la fin de l'expérimentation, le sérum, les cornes utérines, les tibias et les fémurs ont été recueillis pour analyse. Pour l'expérience de perte osseuse induite par LPS, le véhicule (PBS) ou le LPS (sérotype 026: B6, 25mg/kg) ont été injectés par voie sous-cutanée au niveau de la calotte crânienne, une fois par semaine pendant 3 semaines sous anesthésie. Durant ces 3 semaines, les souris ont reçu soit le véhicule (1% d'éthanol, 0,2% hydroxypropylcellulose dans de l'eau distillée) soit la fisétine quotidiennement par gavage. A la fin de l'expérimentation, le sérum, les tibias et les fémurs ont été recueillis pour analyse. Des expériences similaires ont été conduites et arrêtés 24 heures après la première injection de LPS et les fémurs ont été prélevés pour extraction des ARNs et l'analyse en qRT-PCR.

### Analyse de la BMD

L'analyse morphologique osseuse a été effectuée en utilisant un scanner eXplore CT 120 (GE Healthcare, Little Chalfont, Royaume-Uni). Après avoir retiré les tissus mous, les fémurs gauches ont été placés dans un tampon PBS avec 10% de formaldéhyde à 4 ° C pendant une semaine puis analysés. L'acquisition est composée de 360 vues obtenues par incréments de 1 ° recueillies lors une rotation complète du portique, avec une exposition de 20 ms/vue, les paramètres du tube à rayons X étant de 100 kV et 50 mA. Les images ont été reconstruites à l'aide d'un algorithme de faisceau conique modifié avec un voxel isotrope de 0,045 x 0,045 x 0,045 mm³. La tomodensitométrie a été analysée en utilisant le logiciel MicroView ® version 2.3 (GE Healthcare). Un fantôme d'étalonnage hydroxyapatite (SB3, Gamex RMI, WI) a été utilisé pour convertir les niveaux de gris en valeurs de densité osseuse. L'analyse de la densité minérale osseuse a été réalisée au niveau de l'os trabéculaire distal du fémur et la fraction volumique (BVF = volume d'os/volume total) faite dans le centre du fémur sur une région cylindrique (r = 0,7 mm), débutant à 0,1 mm de la plaque de croissance de manière proximale et s'étendant sur 0,32 mm dans la direction proximale. La densité minérale osseuse a été estimée comme une moyenne de niveaux de gris convertis, à l'intérieur de la zone d'intérêt.

### Analyse de la micro-architecture

Pour effectuer une mesure, l'échantillon a été monté sur un plateau tournant pouvant être déplacé automatiquement dans le sens axial (pas angulaire: 0,675°/reconstruction plage angulaire: 186.30°). Un filtre en aluminium (0,5 mm d'épaisseur) a été placé entre la source de rayons X et l'échantillon. La micro-architecture (spongieuse secondaire) a été analysée en utilisant le rayonnement X micro-CT SkyScan 1072 (BRUKER-microCT, Kontich, Belgique). Des photos de 1024 * 1024 pixels ont été obtenues en utilisant les paramètres 37kV et 215µA. Selon les paramètres de la caméra, les pixels mesurent 5.664µm menant à un voxel de 1.817 * 10-7mm³. Le calcul des paramètres histomorphométriques a été réalisé en utilisant les logiciels ctan ® et Nrecon ® (version 1.11. Et 1.6.1.7 respectivement).

### Mesure de l'ostéocalcine sérique

Les niveaux d'ostéocalcine sérique, un marqueur spécifique de la formation osseuse, ont été mesurés en utilisant un test ELISA souris selon le protocole du fabricant (Biomedical Technologies Inc, Stoughton, MA, USA).

### Analyses statistiques

Les résultats sont exprimés en moyenne ± écart-type. Les données ont été analysées soit par analyse de variance Fisher (2 groupes) ou de Newman-Kell (plus de 2 groupes) en utilisant XLSTAT (Addinsoft, Paris, France). Les résultats ont été considérées comme significatifs pour p□0,05.

### 2- Résultats:

### Action synergique de l'association Fisétine/DHA sur l'inhibition de la différenciation des ostéoclastes induite par RANKL in vitro.

La figure 1 montre que la cytokine *Receptor Activator of Nf-κB Ligand* (RANKL) induit la formation de cellules multinucléées positives pour le marquage au Tartrate Resistant Acid Phosphatase (TRAP ou TRACP ; figure 1A) appelées ostéoclastes, conformément à ce qui a été précédemment décrit par notre laboratoire (. Wittrant Y Et al., Bone. 2008 42(6):1122-30). De façon cohérente avec notre expertise, la présence de 5µM de Fisétine et de 50µM d'acide Docosahexaenoique (DHA) inhibe cette induction à hauteur de 45% et 23%, respectivement (735+/-251 ostéoclastes/cm² dans la condition RANKL, 404+/-148 ostéoclastes/cm² dans la condition RANKL+Fisétine, 564+/-259 ostéoclastes/cm² dans la condition RANKL+DHA). De façon innovante, lorsque la Fisétine et le DHA sont combinés, cette association annule presque totalement la formation des ostéoclastes (-97% ; 735+/-251 ostéoclastes/cm² dans la condition RANKL, 24+/-24 ostéoclastes/cm² dans la condition RANKL+Fisétine+DHA). Ainsi, ces données démontrent clairement que la Fisétine et le DHA n'agissent pas seulement de concert, mais en synergie pour limiter la différenciation des cellules responsables de la résorption osseuse. En effet, l'inhibition de la différenciation ostéoclastique par la combinaison Fisétine + DHA est supérieure à la somme des inhibitions des molécules isolées (45 +23 = 68% <97% ; figure 1B). L'analyse statistique montre que seuls les groupes « témoin » et « RANKL+Fisétine+DHA » ne sont pas significativement différents. Dans ce contexte des lettres différentes sont attribuées à des groupes significativement différents (a#b#c#d).

### Action de la Fisétine in vitro sur la différenciation des ostéoblastes.

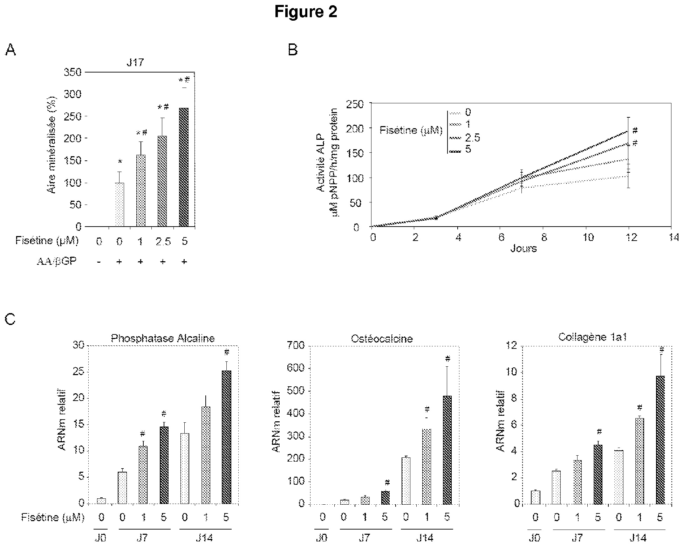

La figure 2 se divise en 3 entités permettant de balayer différentes approches relatives à l'analyse de la différenciation des ostéoblastes primaires. Conformément à notre expertise, la figure 2A montre que la présence de la combinaison Acide Ascorbique (AA) et de β-giycéro-phosphate (βGP) stimule la formation de nodules minéralisés par les ostéoblastes après 17 jours de culture. De façon intéressante, l'addition de Fisétine accroit cette minéralisation de façon dose-dépendante par rapport à la condition « AA+βGP seuls » (+62% pour la condition AA+βGP+Fisétine 1µM ; +105% pour la condition AA+βGP+Fisétine 2,5µM ; +169% pour la condition AA+βGP+Fisétine 5µM). Conformément à l'analyse statistique, (*) signifie significativement différent de la condition « témoin/non induite » et (#) significativement différent de la condition « AA+βGP seuls ». Parallèlement, l'analyse de l'activité Alcaline Phosphatase (figure 2B ; activité ALP), marqueur spécifique des ostéoblastes différenciés, confirme une stimulation par l'addition de Fisétine, également de façon dose-dépendante, par rapport à la condition « AA+βGP seuls » représentée par la ligne en bas en gris 10% (+32% pour la condition AA+âGP+Fisétine 1 µM - ligne grise 25% ; +63% pour la condition AA+βGP+Fisétine 2,5µM - ligne grise 50% ; +87% pour la condition AA+βGP+Fisétine 5µM - ligne noire). Conformément à l'analyse statistique, (#) signifie significativement différent de la condition « AA+βGP seuls ». De façon cohérente, l'analyse du niveau d'expression de trois transcrits (figure 2C) montre une stimulation par la Fisétine de l'expression des marqueurs de la différenciation ostéoblastique par rapport à la condition « AA+βGP seuls » (+84% pour la condition AA+βGP+Fisétine 1µM et +146% pour la condition AA+βGP+Fisétine 5µM à J7 ; +37% pour la condition AA+βGP+Fisétine 1µM et +88% pour la condition AA+βGP+Fisétine 5µM à J14 pour l'expression de la phosphatase alcaline / +63% pour la condition AA+βGP+Fisétine 1 µM et +177% pour la condition AA+βGP+Fisétine 5µM à J7 ; +63% pour la condition AA+βGP+Fisétine 1µM et +131% pour la condition AA+βGP+Fisétine 5µM à J14 pour l'expression de l'ostéocalcine / +34% pour la condition AA+βGP+Fisétine 1 µM et +80% pour la condition AA+βGP+Fisétine 5µM à J7 ; +60% pour la condition AA+βGP+Fisétine 1 µM et +138% pour la condition AA+βGP+Fisétine 5µM à J14 pour l'expression du collagène de type 1). Conformément à l'analyse statistique, (#) signifie significativement différent de la condition « AA+βGP seuls ».

### Action de la Fisétine in vivo sur la perte osseuse induite par ovariectomie

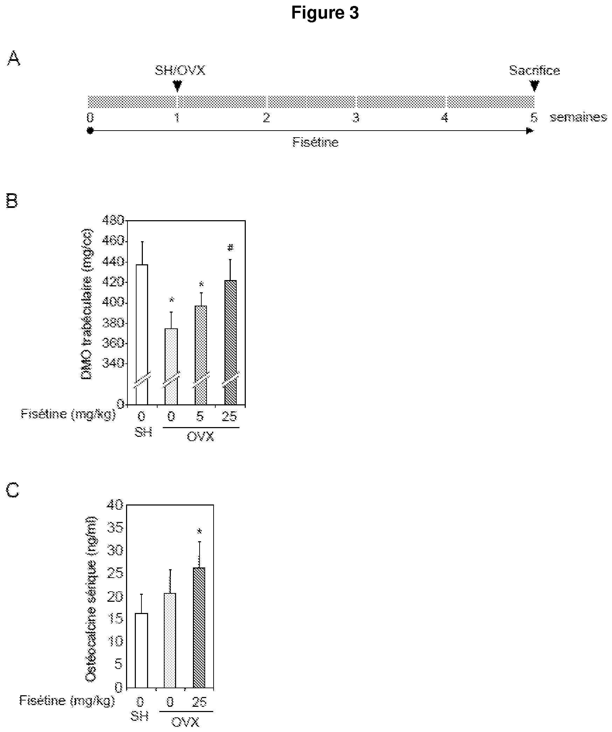

La figure 3 montre le potentiel de prévention sur la perte osseuse de la Fisétine seule ; en accord avec la figure 1, celle-ci laisse envisager le potentiel in vivo de l'association « Fisétine + DHA ». Dans un modèle murin, la perte osseuse est induite par ovariectomie (figure 3A). La perte osseuse induite par l'ovariectomie « OVX » est validée par la mesure de la densité minérale osseuse (-15% par rapport à la condition témoin « SH » ; figure 3B ; DMO trabéculaire). De façon intéressante, l'administration quotidienne de Fisétine permet de prévenir la perte osseuse induite par l'ovariectomie (seulement -4% pour la condition Fisétine 25mg/kg par rapport à la condition témoin « SH »). Conformément à l'analyse statistique, (*) signifie significativement différent de la condition témoin «SH» et (#) significativement différent de la condition « OVX sans Fisétine». L'effet préventif de la fisétine sur la perte osseuse est également validé par la mesure des paramètres architecturaux. Comme illustré dans le tableau ci-dessous, le volume osseux/volume total, le nombre de trabécules et l'épaisseur des trabécules sont significativement plus importants chez les animaux OVX + Fisétine 25 mg/kg par rapport aux animaux OVX (augmentation respective de 20,37 %, 16,31 % et 3,55 %).

| | OVX | OVX + Fisétine 25mg/kg | valeur p |
|---|---|---|---|
| Volume osseux/Volume total (%) | 13.40 ± 1.74 | 16.13 ± 0.96 | 0.034 |
| Nombre de trabécules (mm⁻¹) | 1.90 ± 0.22 | 2.21 ± 0.12 | 0.046 |
| Epaisseur des trabécules (µm) | 70.35 ± 1.295 | 72.85 ± 1.563 | 0.049 |
| Espace inter-trabéculaire (µm) | 285.68 ± 54,69 | 265.50 ± 29.41 | 0.540 |

De façon cohérente, le taux sérique d'ostéocalcine est également stimulé par l'administration quotidienne de Fisétine à 25mg/kg traduisant ainsi une augmentation de l'activité de synthèse de la matrice osseuse par les ostéoblastes (+61% par rapport à la condition témoin « SH » figure 3C ; Ostéocalcine sérique). Conformément à l'analyse statistique, (*) signifie significativement différent de la condition « SH ».

## Revendications

1. Association comprenant au moins
(i) la fisétine, et
(ii) l'acide docosahexaénoïque (DHA), ou l'un de ses dérivés choisis sous forme d'un glycéride ou d'un phospholipide,
pour son utilisation pour le traitement et/ou la prévention de troubles associés à une perte osseuse et/ou à un déséquilibre du rapport entre la formation et la résorption osseuse.

2. Association, pour son utilisation selon la revendication 1, **caractérisée en ce que** les dérivés de la DHA sous forme d'un glycéride est sous forme de mono-, di- ou triglycérides.

3. Association, pour son utilisation selon l'une quelconque des revendications 1 ou 2, pour inhiber la résorption osseuse et/ou pour stimuler la formation osseuse et/ou pour prévenir ou traiter l'ostéopénie, la raréfaction osseuse, la fragilisation du tissu osseux, ou l'ostéoporose.

4. Association, pour son utilisation selon l'une des revendications 1 à 3, **caractérisée en ce que** les troubles associés à une perte osseuse, et/ou à un déséquilibre du rapport entre la formation et la résorption osseuse sont choisis parmi la perte osseuse associée au vieillissement et/ou à la ménopause, la perte osseuse consécutive à certaines pathologies ou à la prise de médicaments, l'ostéolyse observée à proximité d'une prothèse, la perte osseuse associée aux pathologies dentaires ou parodontales, la maladie de Paget, les maladies tumorales ostéolytiques, l'hypercalcémie due à un cancer, et les fractures.

5. Association, pour son utilisation selon l'une des revendications précédentes, **caractérisée en ce que** la fisétine et/ou le DHA, ou l'un de ses dérivés, est sous forme d'extrait naturel en contenant.

6. Association, pour son utilisation selon l'une des revendications précédentes, **caractérisée en ce qu'**elle contient un ratio fisétine / DHA allant de 100/1 à 1/100.

7. Association pour son utilisation selon l'une des revendications précédentes, **caractérisée en ce que** (i) la fisétine est administrée à une dose de 0,01 à 1 g/jour et (ii) le DHA, ou l'un de ses dérivés, est administré à une dose de 0,01 à 3 g/jour.

8. Association, pour son utilisation selon l'une des revendications 1 à 7, **caractérisée en ce qu'**elle est sous forme d'un produit de combinaison comprenant au moins :
a) une quantité efficace de fisétine et
b) une quantité efficace de DHA ou de l'un de ses dérivés;
pour une utilisation séquentielle, séparée ou simultanée dans le temps.

9. Association, pour son utilisation selon l'une des revendications 1 à 8, **caractérisée en ce qu'**elle est formulée sous forme d'au moins une composition comprenant au moins un excipient physiologiquement acceptable.

10. Association, pour son utilisation selon la revendication 9, **caractérisée en ce qu'**elle est sous forme d'au moins deux compositions a) et b) comprenant respectivement, au moins une quantité efficace (i) de fisétine et au moins une quantité efficace (ii) de DHA, ou de l'un de ses dérivés ;
l'une au moins desdites compositions comprenant au moins un excipient physiologiquement acceptable.

11. Composition comprenant une association pour son utilisation selon l'une des revendications 9 ou 10, **caractérisée en ce qu'**elle est adaptée à une administration par voie orale.

12. Composition comprenant une association pour son utilisation selon la revendication 11, **caractérisée en ce que** ladite composition se présente sous forme de gélule, poudre, lyophilisat, granule, comprimé, capsule, sirop, infusion, jus, ou macérât, extrait sec, extrait mou, extrait hydro-alcoolique.

13. Composition comprenant une association pour son utilisation selon la revendication 12, **caractérisée en ce que** ladite composition se présente sous forme de produit laitier, de dérivé de laits végétaux, de produit dérivé de fruits ou légumes, de produit dérivé de poissons, de produit céréalier, de corps gras, de plat cuisiné ou de boisson.

14. Composition comprenant une association, pour son utilisation selon l'une des revendications précédentes, **caractérisée en ce qu'**elle contient en outre au moins un composé choisi parmi le groupe constitué par les sels de calcium inorganiques, les composés contenant du calcium, les vitamines, les éléments minéraux, les fibres, les prébiotiques, les composés source d'énergie.

## Patentansprüche

1. Kombination, die mindestens Folgendes umfasst
(i) Fisetin, und
(ii) Docosahexaensäure (DHA), oder eines von deren Derivaten, wobei diese derart ausgewählt sind, dass es in Form eines Glycerids oder eines Phospholipids vorliegen,
wobei sie dazu verwendet wird, Störungen im Zusammenhang mit einem Knochenschwund und/oder einem Ungleichgewicht des Verhältnisses zwischen der Neubildung und dem Abbau von Knochenmasse zu behandeln und/oder zu verhüten.

2. Kombination für eine Verwendung nach Anspruch 1, **dadurch gekennzeichnet, dass** die DHA-Derivate in Glyceridform als Mono-, Di- oder Triglyceride vorliegen.

3. Kombination für eine Verwendung nach einem beliebigen der Ansprüche 1 oder 2, um den Knochenabbau zu hemmen und/oder um die Knochenneubildung zu stimulieren und/oder um Osteopenie, Knochenverlust, eine erhöhte Verletzungsanfälligkeit des Knochengewebes oder Osteoporose zu verhüten und zu behandeln.

4. Kombination zur Verwendung nach einem der Ansprüche 1 bis 3, **dadurch gekennzeichnet, dass** die Störungen im Zusammenhang mit einem Knochenschwund und/oder einem Ungleichgewicht des Verhältnisses zwischen der Neubildung und dem Abbau von Knochenmasse aus dem Knochenschwund im Zusammenhang mit der Alterung und/oder der Menopause, dem Knochenschwund infolge bestimmter Pathologien oder der Einnahme von Medikamenten, der Osteolyse, wie sie in der Nähe einer Prothese zu beobachten ist, dem Knochenschwund im Zusammenhang mit Erkrankungen der Zähne oder des Zahnhalteapparats, der Osteodystrophie deformans, den osteolytischen tumorartigen Krankheiten, der krebsbedingten Hypercalcämie sowie Knochenbrüchen ausgewählt sind.

5. Kombination zur Verwendung nach einem der vorhergehenden Ansprüche, **dadurch gekennzeichnet, dass** das Fisetin und/oder die DHA, oder eines von deren Derivaten, in Form eines natürlichen Extrakts vorliegt, in welchem es/sie enthalten ist/sind.

6. Kombination zur Verwendung nach einem der vorhergehenden Ansprüche, **dadurch gekennzeichnet, dass** sie Fisetin / DHA in einem Verhältnis enthält, das im Bereich von 100/1 bis 1/100 liegt.

7. Kombination zur Verwendung nach einem der vorhergehenden Ansprüche, **dadurch gekennzeichnet, dass** (1) Fisetin in einer Dosis von 0,01 bis 1 g/Tag verabreicht wird und (ii) DHA, oder eines von deren Derivaten, in einer Dosis von 0,01 bis 3 g/Tag verabreicht wird.

8. Kombination zur Verwendung nach einem der Ansprüche 1 bis 7, **dadurch gekennzeichnet, dass** sie in Form eines Kombinationspräparats vorliegt, das mindestens Folgendes umfasse:
a) eine wirksame Menge an Fisetin und
b) eine wirksame Menge an DHA oder an einem ihrer Derivate;
für eine Verwendung, die zeitlich gesehen nacheinander, getrennt voneinander oder gleichzeitig erfolgt.

9. Kombination zur Verwendung nach einem der Ansprüche 1 bis 8, **dadurch gekennzeichnet, dass** sie in Form mindestens einer Zusammensetzung formuliert ist, die mindestens einen physiologisch unbedenklichen Hilfsstoff umfasst.

10. Kombination zur Verwendung nach Anspruch 9, **dadurch gekennzeichnet, dass** sie in Form von mindestens zwei Zusammensetzungen a) und b) vorliegt, die jeweils zumindest eine wirksame Menge (i) an Fisetin beziehungsweise zumindest eine wirksame Menge (ii) an DHA, oder an einem von deren Derivaten, umfassen; wobei mindestens eine der Zusammensetzungen mindestens einen physiologisch unbedenklichen Hilfsstoff umfasst.

11. Zusammensetzung, die eine Kombination zur Verwendung gemäß einem der Ansprüche 9 oder 10 umfasst, **dadurch gekennzeichnet, dass** sie für eine Verabreichung auf oralem Wege geeignet ist.

12. Zusammensetzung, die eine Kombination zur Verwendung nach Anspruch 11 umfasst, **dadurch gekennzeichnet, dass** die Zusammensetzung als Gelatinekapsel, Pulver, Lyophilisat, Granulat, Tablette, Kapsel, Sirup, Aufguss, Saft oder als Mazerat, Trockenextrakt, Spissum-Extrakt, wässrigalkoholischer Extrakt vorliegt.

13. Zusammensetzung, die eine Kombination zur Verwendung nach Anspruch 12 umfasst, **dadurch gekennzeichnet, dass** die Zusammensetzung in Form eines Milchprodukts, eines Derivates milchartiger pflanzlicher Erzeugnisse, eines Produkts auf Basis von Obst oder Gemüse, eines Produkts auf Basis von Fisch, eines Getreideprodukts, eines Fettstoffs, eines Fertiggerichts oder eines Getränks vorliegt.

14. Zusammensetzung, die eine Kombination zur Verwendung nach einem der vorhergehenden Ansprüche umfasst, **dadurch gekennzeichnet, dass** sie darüber hinaus mindestens eine Verbindung enthält, die aus der Gruppe ausgewählt ist, welche aus den anorganischen Calciumsalzen, den calciumhaltigen Verbindungen, den Vitaminen, den Mineralstoffelementen, den Nahrungsfasern, den Präbiotika, die energieliefernden Verbindungen besteht.

## Claims

1. A combination comprising at least
(i) fisetin, and
(ii) docosahexaenoic acid (DHA), or a derivative thereof, selected in the form of a glyceride or a phospholipid,
for use in the treatment and/or the prevention of disorders associated with a bone loss and/or an imbalance of the ratio between the bone formation and resorption.

2. The combination for use according to claim 1, **characterized in that** the DHA derivatives in the form of a glyceride are in the form of mono-, di- or triglycerides.

3. The combination for use according to any one of claims 1 or 2, for inhibiting bone resorption and/or stimulating bone formation and/or preventing or treating osteopenia, rarefaction of bone, weakening of bone tissue, or osteoporosis.

4. The combination for use according to one of claims 1 to 3, **characterized in that** the disorders associated with a bone loss and/or an imbalance of the ratio between the bone formation and resorption are selected from bone loss associated with ageing and/or menopause, bone loss subsequent to certain pathologies or drug intake, osteolysis observed near a prosthesis, bone loss associated with dental or periodontal pathologies, Paget's disease, osteolytic tumor diseases, hypercalcemia due to a cancer, and fractures.

5. The combination for use according to one of the preceding claims, **characterized in that** the fisetin and/or DHA, or a derivative thereof, is in the form of natural extract containing the same.

6. The combination for use according to one of the preceding claims, **characterized in that** it contains a fisetin / DHA ratio of from 100/1 to 1/100.

7. The combination for use according to one of the preceding claims, **characterized in that** (i) the fisetin is administered at a dose of 0.01 to 1 g/day and (ii) the DHA, or a derivative thereof, is administered at a dose of 0.01 to 3 g/day.

8. The combination for use according to one of claims 1 to 7, **characterized in that** it is in the form of a combination product comprising at least:
a) an effective amount of flsetin, and
b) an effective amount or DHA or a derivative thereof;
for sequential, separate or simultaneous use over time.

9. The combination for use according to one of claims 1 to 8, **characterized in that** it is formulated in the form of at least one composition comprising at least one physiologically acceptable excipient.

10. The combination for use according to claim 9, **characterized in that** it is in the form of at least two compositions a) and b) respectively comprising at least an effective amount (i) of fisetin and at least an effective amount (ii) of DHA, or a derivative thereof;
at least one of said compositions comprising at least one physiologically acceptable excipient.

11. A composition comprising a combination for use according to one of claims 9 or 10, **characterized in that** it is suitable for oral administration.

12. The composition comprising a combination for use according to claim 11, **characterized in that** said composition is in the form of hard capsule, powder, lyophilisate, granule, tablet, capsule, syrup, infusion, juice, or macerate, dry extract, soft extract, hydroalcoholic extract.

13. The composition comprising a combination for use according to claim 12, **characterized in that** said composition is in the form of dairy product, vegetal milk derivative, product derived from fruits or vegetables, product derived from fish, cereal product, fat, ready meal or drink.

14. The composition comprising a combination for use according to one of the preceding claims, **characterized in that** it further contains at least one compound selected from the group consisting of inorganic calcium salts, calcium containing compounds, vitamins, mineral elements, fibers, prebiotics, energy source compounds.
